(19) Europäisches Patentamt
European Patent Office
Office européen des brevets

(11) EP 1 337 465 B1

(12) # EUROPEAN PATENT SPECIFICATION

(45) Date of publication and mention of the grant of the patent:
**13.06.2007 Bulletin 2007/24**

(21) Application number: **01980793.2**

(22) Date of filing: **31.10.2001**

(51) Int Cl.:
***C01B 39/06*** (2006.01) ***C01B 39/48*** (2006.01)
***B01J 29/70*** (2006.01) ***B01J 29/87*** (2006.01)
***C10G 45/64*** (2006.01) ***C10G 29/20*** (2006.01)
***C07C 5/27*** (2006.01) ***C07C 2/66*** (2006.01)
***C07C 6/12*** (2006.01)

(86) International application number:
**PCT/IB2001/002044**

(87) International publication number:
**WO 2002/036489 (10.05.2002 Gazette 2002/19)**

(54) **UZM-5, UZM-5P AND UZM-6; CRYSTALLINE ALUMINOSILICATE ZEOLITES AND PROCESSES USING THE SAME**

UZM-5, UZM-5P UND UZM-6 TYP KRISTALLINE ALUMINOSILIKAT-ZEOLITHE UND SIE ANWENDENDE VERFAHREN

UZM-5, UZM-5P ET UZM-6; ZEOLITES ALUMINO-SILICATES CRISTALLINS ET PROCEDES D'UTILISATION DE CES DERNIERS

(84) Designated Contracting States:
**AT BE CH CY DE DK ES FI FR GB GR IE IT LI LU MC NL PT SE TR**

(30) Priority: **03.11.2000 US 705860**
**03.11.2000 US 706285**
**03.11.2000 US 705839**

(43) Date of publication of application:
**27.08.2003 Bulletin 2003/35**

(73) Proprietor: **UOP LLC**
**Des Plaines, IL 60017-5017 (US)**

(72) Inventors:
- **MOSCOSO, Jaime G., c/o UOP LLC Des Plaines, IL 60017-5017 (US)**
- **LEWIS, Gregory J., c/o UOP LLC Des Plaines, IL 60017-5017 (US)**
- **MILLER, Mark A., c/o UOP LLC Des Plaines, IL 60017 (US)**
- **JAN, Deng-Yang, c/o UOP LLC Des Plaines, IL 60017 (US)**
- **PATTON, Robert L., c/o UOP LLC Des Plaines, IL 60017 (US)**
- **ROHDE, Lisa M., c/o UOP LLC Des Plaines, IL 60017 (US)**
- **CHEN, Qianjin, c/o UOP LLC Des Plaines, IL 60017 (US)**

(74) Representative: **Hayes, Adrian Chetwynd et al**
**Boult Wade Tennant,**
**Verulam Gardens**
**70 Gray's Inn Road**
**London WC1X 8BT (GB)**

(56) References cited:
**EP-A- 0 074 652**
**EP-A- 0 231 018**
**EP-A- 0 463 768**
**EP-A- 0 818 417**
**EP-A- 0 825 152**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 337 465 B1

## Description

BACKGROUND OF THE INVENTION

Zeolites are crystalline aluminosilicate compositions which are microporous and which have a three-dimensional oxide framework formed from corner sharing $AlO_2$ and $SiO_2$ tetrahedra. Numerous zeolites, both naturally occurring and synthetically prepared are used in various industrial processes. Zeolites are characterized by having pore openings of uniform dimensions, having a significant ion exchange capacity, and being capable of reversibly desorbing an adsorbed phase which is dispersed throughout the internal voids of the crystal without significantly displacing any atoms which make up the permanent zeolite crystal structure.

The number of synthetic zeolites is well over a hundred as evidenced by the Atlas of Zeolite Structure Types published by the International Zeolite Association (IZA). As is well known, zeolites are distinguished from each other on the basis of their composition, crystal structure and adsorption properties. One method commonly used in the art to distinguish zeolites is x-ray diffraction.

Applicants have synthesized a family of crystalline zeolitic compositions which have unique x-ray diffraction patterns and have an empirical formula on an anhydrous basis in terms of molar ratios of:

$$M_m^{n+} R_r^{p+} Al_{(1-x)} E_x Si_y O_z$$

where M is at least one exchangeable cation selected from the group consisting of alkali and alkaline earth metals, "m" is the mole ratio of M to (Al + E) and varies from 0 to 1.2, R is a nitrogen-containing organic cation selected from the group consisting of quaternary ammonium ions, protonated amines, protonated diamines, protonated alkanolamines, quaternary alkanolammonium ions, diquaternary ammonium ions, and mixtures thereof, "r" is the mole ratio of R to (Al+ E) and has a value of 0.25 to 3.0, E is an element selected from the group consisting of Ga, Fe, In, Cr, and B, "x" is the mole fraction of E and varies from 0 to 0.5, "n" is the weighted average valence of M and has a value of +1 to +2, "p" is the weighted average valence of R and has a value of +1 to +2, "y" is the mole ratio of Si to (Al + E) and varies from 5 to 12, and "z" is the mole ratio of O to (Al + E) and has a value determined by the equation:

$$z = (m \bullet n + r \bullet p + 3 + 4 \bullet y)/2$$

Specific members of this family of zeolites are: UZM-5, UZM-5P and UZM-6. These zeolites can catalyze various hydrocarbon conversion processes such as alkylation of benzene and isomerization of xylene.

DETAILED DESCRIPTION OF THE INVENTION

Applicants have synthesized a new family of zeolites. In its assynthesized form, this family of zeolites has a composition on an anhydrous basis that is represented by the formula:

$$M_m^{n+} R_r^{p+} Al_{(1-x)} E_x Si_y O_z$$

M is an exchangeable cation and is selected from the group consisting of alkali and alkaline earth metals. Specific examples of the M cations include but are not limited to lithium, sodium, potassium, cesium, strontium, calcium, magnesium, barium and mixtures thereof. The value of "m" which is the mole ratio of M to (Al + E) varies from 0 to 1.2. R is a nitrogen containing organic cation and is selected from the group consisting of protonated amines, protonated diamines, protonated alkanolamines, quaternary ammonium ions, diquaternary ammonium ions, quaternized alkanolammonium ions and mixtures thereof. The value of "r" which is the mole ratio of R to (Al + E) varies from 0.25 to 3.0. The value of "n" which is the weighted average valence of M varies from +1 to +2. The value of "p", which is the average weighted valence of the organic cation has a value from +1 to +2. E is an element which is present in the framework and is selected from the group consisting of gallium, iron; boron chromium, indium and mixtures thereof. The value of "x" which is the mole fraction of E varies from 0 to 0.5. The ratio of silicon to (Al+E) is represented by "y" which varies from 5 to 12, while the mole ratio of O to (Al+E) is represented by "z" and " has a value given by the equation:

$$z = (m \bullet n + r \bullet p + 3 + 4 \bullet y)/2$$

When M is only one metal, then the weighted average valence is the valence of that one metal, i.e. +1 or +2. However, when more than one M meta is present, the total amount of:

$$M_m^{n+} = M_{m1}^{(n1)+} + M_{m2}^{(n2)+} + M_{m3}^{(n3)+} + .....$$

and the weighted average valence "n" is given by the equation:

$$n = \frac{m_1 \bullet n_1 + m_2 \bullet n_2 + m_3 \bullet n_3 + \cdots}{m_1 + m_2 + m_3 \cdots}$$

Similarly when only one R organic cation is present, the weighted average valence is the valence of the single R cation, i.e., +1 or +2. When more than one R cation is present, the total amount of R is given by the equation:

$$R_r^{p+} = R_{r1}^{(p1)+} + R_{r2}^{(p2)+} + R_{r3}^{(p3)+}$$

and the weighted average valence "p" is given by the equation:

$$p = \frac{p_1 \bullet r_1 + p_2 \bullet r_2 + p_3 \bullet r_3 + \cdots}{r_1 + r_2 + r_3 + \cdots}.$$

These aluminosilicate zeolites, are prepared by a hydrothermal crystallization of a reaction mixture prepared by combining reactive sources of R, aluminum, optionally E and/or M and silicon in aqueous media. Accordingly, the aluminum sources include, but are not limited to, aluminum alkoxides, precipitated alumina, aluminum hydroxide, aluminum salts and aluminum metal. Specific examples of aluminum alkoxides include, but are not limited to aluminum ortho-sec-butoxide, and aluminum ortho-isopropoxide. Sources of silica include but are not limited to tetraethylorthosilicate, fumed silicas, precipitated silicas and colloidal silica. Sources of the M metals include but are not limited to the halide salts, nitrate salts, acetate salts, and hydroxides of the respective alkali or alkaline earth metals. Sources of the E elements include but are not limited to alkali borates, boric acid, precipitated gallium oxyhydroxide, gallium sulfate, ferric sulfate, ferric chloride, chromium chloride, chromium nitrate, indium chloride and indium nitrate. When R is a quaternary ammonium cation, the sources include without limitation the hydroxide, and halide compounds. Specific examples include without limitation tetramethylammonium hydroxide, tetraethylammonium hydroxide, hexamethonium bromide, tetramethylammonium chloride, methyltriethylammonium hydroxide. R may also be neutral amines, diamines, and alkanolamines. Specific examples are triethanolamine, triethylamine, and N,N,N',N'tetramethyl-1,6-hexanediamine.

The reaction mixture containing reactive sources of the desired components can be described in terms of molar ratios of the oxides by the formula:

$$aM_{2/n}O:bR_{2/p}O:(1-c)Al_2O_3:cE_2O_3:dSiO_2:eH_2O$$

where "a" is the mole ratio of the oxide of M and has a value of 0 to 2, "b" is the mole ratio of the oxide of R and has a value of 1.5 to 30, "d" is the mole ratio of silica and has a value of 5 to 30, "c" is the mole ratio of the oxide of E and has a value from 0 to 0.5, and "e" is the mole ratio of water and has a value of 30 to 6000. The reaction mixture is now reacted at reaction conditions including a temperature of 100°C to 175°C and preferably from 140°C to 160°C for a period of 12 hours to 14 days and preferably for a time of 2 days to 5 days in a sealed reaction vessel under autogenous pressure. After crystallization is complete, the solid product is isolated from the heterogeneous mixture by means such

as filtration or centrifugation, and then washed with de-ionized water and dried in air at ambient temperature up to 100°C.

As synthesized, the zeolites will contain some of the exchangeable or charge balancing cations in its pores. These exchangeable cations can be exchanged for other cations, or in the case of organic cations, they can be removed by heating under controlled conditions. All of these methods are well known in the art.

The crystalline zeolites are characterized by a three-dimensional framework structure of at least $SiO_2$ and $AlO_2$ tetrahedral units. These zeolites are further characterized by their unique x-ray diffraction pattern. The x-ray diffraction pattern has at least two peaks: one peak at a *d*-spacing of 3.9±0.12Å and one peak at a *d*-spacing of 8.6±0.20Å. To allow for ready reference, the different structure types and compositions of crystalline zeolites have been given arbitrary designation of UZM-h, where "h" is an integer starting at one and where for example "1" represents a framework of structure type "1". That is one or more zeolitic composition with different empirical formulas can have the same structure type "h", e.g. "1".

In this respect, the following species can be identified by their x-ray diffraction patterns which have at least the *d*-spacing and relative intensities set forth in Tables A to C.

Table A

| UZM-5 | | |
|---|---|---|
| 2-θ | d(Å) | $I/I_o\%$ |
| 6.31- 5.89 | 14.00-15.00 | w-m |
| 7.96- 7.58 | 11.10 - 11.65 | m-s |
| 10.40- 10.01 | 8.50 - 8.83 | w-m |
| 12.11 - 11.59 | 7.30 - 7.63 | m |
| 16.10- 15.53 | 5.50 - 5.70 | m-vs |
| 19.28 - 18.55 | 4.60 - 4.78 | w-m |
| 22.26 - 21.60 | 3.99 - 4.11 | m |
| 23.20 - 22.43 | 3.83 - 3.96 | w-s |
| 24.16 - 23.33 | 3.68 - 3.81 | vs |
| 30.48 - 29.55 | 2.93 - 3.02 | w-m |
| 31.94 - 30.92 | 2.80 - 2.89 | w-m |
| 44.83 - 43.47 | 2.02 - 2.08 | w |

Table B

| UZM-5P | | |
|---|---|---|
| 2-θ | D(Å) | $I/I_o\%$ |
| 6.31 -5.19 | 14.00- 17.00 | w-vs |
| 7.96-7.56 | 11.10-11.70 | w-m |
| 10.52-10.04 | 8.40-8.80 | m-s |
| 16.56 -15.67 | 5.35 - 5.65 | w-m |
| 19.49 - 18.87 | 4.55 - 4.70 | w - m |
| 23.52 - 22.09 | 3.78 - 4.02 | w - vs |
| 24.03 - 23.39 | 3.70 - 3.80 | w - vs |
| 30.81 - 29.76 | 2.90 - 3.00 | w - m |
| 31.94-30.81 | 2.80-2.90 | w - m |
| 45.30-43.04 | 2.00 - 2.10 | w - m |

Table C

| UZM-6 | | |
|---|---|---|
| 2-θ | D(Å) | $I/I_o$% |
| 6.31 - 5.89 | 14.00-15.00 | w-m |
| 7.96 - 7.58 | 11.10 - 11.65 | m-s |
| 10.40- 10.01 | 8.50 - 8.83 | w-m |
| 12.11 - 11.59 | 7.30 - 7.63 | m |
| 16.10 - 15.53 | 5.50 - 5.70 | m-vs |
| 19.28 - 18.55 | 4.60 - 4.78 | w-m |
| 22.26 - 21.60 | 3.99 - 4.11 | m |
| 23.20 - 22.43 | 3.92 - 4.00 | m-vs |
| 24.16 - 23.33 | 3.83 - 3.96 | w-s |
| 30.48 - 29.55 | 3.68 - 3.81 | s-vs |
| 31.94 - 30.92 | 2.80 - 2.89 | m |
| 44.83 - 43.47 | 2.02 - 2.08 | w |

The zeolites of this invention are capable of separating mixtures of molecular species based on the molecular size (kinetic diameter) or on the degree of polarity of the molecular species. When the separation of molecular species is based on molecular size, separation is accomplished by the smaller molecular species entering the intracrystalline void space while excluding larger species. The kinetic diameters of various molecules such as oxygen, nitrogen, carbon dioxide, carbon monoxide are provided in D.W. Breck, Zeolite Molecular Sieves, John Wiley and Sons (1974) p. 636.

The crystalline microporous compositions of the present invention either as synthesized or after calcination can be used as catalysts or catalyst supports in hydrocarbon conversion processes. Hydrocarbon conversion processes are well known in the art and include cracking, hydrocracking, alkylation of both aromatics and isoparaffins, isomerization, polymerization, reforming, dewaxing, hydrogenation, dehydrogenation, transalkylation, dealkylation, hydration, dehydration, hydrotreating, hydrodenitrogenation, hydrodesulfurization, methanation and syngas shift process. Specific reaction conditions and the types of feeds which can be used in these processes are well known in the art. Preferred hydrocarbon conversion processes are alkylation of aromatics and isomerization of xylenes.

Other reactions may be catalyzed by these crystalline microporous compositions, including base-catalyzed side chain alkylation of alkylaromatics, aldol-condensations, olefin double bond isomerization and isomerization of acetylenes, alcohol dehydrogenation, and olefin dimerization, oligomerization and conversion of alcohol to olefins. Suitably ion exchanged forms of these materials can catalyze the reduction of $NO_x$ to $N_2$ in automotive and industrial exhaust streams. Some of the reaction conditions and types of feeds that can be used in these processes are set forth in US-A-5,015,796 and in H. Pines, THE CHEMISTRY OF CATALYTIC HYDROCARBON CONVERSIONS, Academic Press (1981) pp. 123-154 and references contained therein.

Although the zeolites can be used alone, it is preferable that the zeolite be mixed with a binder for convenient formation of catalyst particles in a proportion of 5 to 100 mass % zeolite and 0 to 95 mass-% binder, with the zeolite preferably comprising from 10 to 90 mass-% of the composite. The binder should preferably be porous, have a surface area of 5 to 800 $m^2/g$, and relatively refractory to the conditions utilized in the hydrocarbon conversion process. Nonlimiting examples of binders are aluminas, titania, zirconia, zinc oxide, magnesia, boria, silica-alumina, silica-magnesia, chromia-alumina, alumina-boria, silica-zirconia, silica, silica gel, and clays. Preferred binders are amorphous silica and alumina, including gamma-, eta-, and theta-alumina, with gamma- and eta-alumina being especially preferred.

The zeolite with or without a binder can be formed into various shapes such as pills, pellets, extrudates, spheres, etc. Preferred shapes are extrudates and spheres. Extrudates are prepared by conventional means which involves mixing of zeolite either before or after adding metallic components, with the binder and a suitable peptizing agent to form a homogeneous dough or thick paste having the correct moisture content to allow for the formation of extrudates with acceptable integrity to withstand direct calcination. The dough then is extruded through a die to give the shaped extrudate. A multitude of different extrudate shapes are possible, including, but not limited to, cylinders, cloverleaf, dumbbell and symmetrical and asymmetrical polylobates. It is also within the scope of this invention that the extrudates may be further shaped to any desired form, such as spheres, by any means known to the art.

Spheres can be prepared by the well known oil-drop method which is described in US-A-2,620,314 which is incorporated by reference. The method involves dropping a mixture of zeolite, and for example, alumina sol, and gelling agent into an oil bath maintained at elevated temperatures. The droplets of the mixture remain in the oil bath until they set and form hydrogel spheres. The spheres are then continuously withdrawn from the oil bath and typically subjected to specific aging treatments in oil and an ammoniacal solution to further improve their physical characteristics. The resulting aged and gelled particles are then washed and dried at a relatively low temperature of 50-200°C and subjected to a calcination procedure at a temperature of 450-700°C for a period of 1 to 20 hours. This treatment effects conversion of the hydrogel to the corresponding alumina matrix.

A platinum-group metal, including one or more of platinum, palladium, rhodium, ruthenium, osmium, and iridium, is an optional component of the present catalyst but necessary for isomerization and alkylation. The preferred platinum-group metal is platinum. The platinum-group metal component may exist within the final catalyst composite as a compound such as an oxide, sulfide, halide, oxysulfide, etc., or as an elemental metal or in combination with one or more other ingredients of the catalyst composite. It is believed that the best results are obtained when substantially all the platinum-group metal component exists in a reduced state. The platinum-group metal component generally comprises from 0.01 to 5 mass-% and preferably from 0.1 to 2% of the final catalyst composite, calculated on an elemental basis.

The platinum-group metal component may be incorporated into the catalyst composite in any suitable manner. One method of preparing the catalyst involves the utilization of a water-soluble, decomposable compound of a platinum-group metal to impregnate the calcined sieve/binder composite. Alternatively, a platinum-group metal compound may be added at the time of compositing the zeolite and binder. Yet another method of effecting a suitable metal distribution is by compositing the metal component with the binder prior to co-extruding the zeolite and binder. Complexes of platinum-group metals which may be employed according to the above or other known methods include chloroplatinic acid, chloropalladic acid, ammonium chloroplatinate, bromoplatinic acid, platinum trichloride, platinum tetrachloride hydrate, platinum dichlorocarbonyl dichloride, tetramine platinic chloride, dinitrodiaminoplatinum, sodium tetranitroplatinate (II), palladium chloride, palladium nitrate, palladium sulfate, diamminepalladium (II) hydroxide, tetramminepalladium (II) chloride, and the like.

It is within the scope of the present invention that the catalyst composite may contain other metal components known to modify the effect of the platinum-group metal component. Such metal modifiers may include rhenium, tin, germanium, lead, cobalt, nickel, indium, gallium, zinc, uranium, dysprosium, thallium, and mixtures thereof. Catalytically effective amounts of such metal modifiers may be incorporated into the catalyst by any means known in the art to effect a homogeneous or stratified distribution.

The catalyst composite of the present invention may contain a halogen component. The halogen component may be either fluorine, chlorine, bromine or iodine or mixtures thereof, with chlorine being preferred. The halogen component is generally present in a combined state with the inorganic-oxide support. The optional halogen component is preferably well dispersed throughout the catalyst and may comprise from more than 0.2 to 15 wt.%, calculated on an elemental basis, of the final catalyst. The halogen component may be incorporated in the catalyst composite in any suitable manner, either during the preparation of the inorganic-oxide support or before, while or after other catalytic components are incorporated.

The catalyst composite is dried at a temperature of from 100° to 320°C for a period of from 2 to 24 or more hours and, usually, calcined at a temperature of from 400°to 650°C in an air atmosphere for a period of from 1 to 10 hours until the metallic compounds present are converted substantially to the oxide form. If desired, the optional halogen component may be adjusted by including a halogen or halogen-containing compound in the air atmosphere.

The resultant calcined composite optimally is subjected to a substantially water-free reduction step to insure a uniform and finely divided dispersion of the optional metallic components. The reduction optionally may be effected *in situ.* Substantially pure and dry hydrogen (i.e., less than 20 vol. ppm $H_2O$) preferably is used as the reducing agent in this step. The reducing agent contacts the catalyst at conditions, including a temperature of from 200° to 650°C and for a period of from 0.5 to 10 hours, effective to reduce substantially all of the Group VIII metal component to the metallic state. In some cases the resulting reduced catalyst composite may also be beneficially subjected to presulfiding by a method known in the art to incorporate in the catalyst composite from 0.05 to 1.0 mass-% sulfur calculated on an elemental basis.

The feedstock to aromatics isomerization comprises isomerizable alkylaromatic hydrocarbons of the general formula $C_6H_{(6-n)}R_n$, where n is an integer from 1 to 5 and R is $CH_3$, $C_2H_5$, $C_3H_7$, or $C_4H_9$, in any combination and including all the isomers thereof to obtain more valuable isomers of the alkylaromatic. Suitable alkylaromatic hydrocarbons include without limitation ortho-xylene, meta-xylene, para-xylene, ethylbenzene, ethyltoluenes, tri-methylbenzenes, di-ethylbenzenes, tri-ethyl-benzenes, methylpropylbenzenes, ethylpropylbenzenes, di-isopropylbenzenes, and mixtures thereof.

Isomerization of a $C_8$-aromatic mixture containing ethylbenzene and xylenes is a particularly preferred application for the zeolites of the invention. Generally such mixture will have an ethylbenzene content in the approximate range of 5 to 50 mass-%, an ortho-xylene content in the approximate range of 0 to 35 mass-%, a meta-xylene content

in the approximate range of 20 to 95 mass-% and a para-xylene content in the approximate range of 0 to 15 mass-%. It is preferred that the aforementioned $C_8$ aromatics comprise a non-equilibrium mixture, i.e., at least one $C_8$-aromatic isomer is present in a concentration that differs substantially (defined herein as a difference of at least 5 mass-% of the total $C_8$ aromatics) from the thermodynamic equilibrium concentration of that isomer at isomerization conditions. Usually the non-equilibrium mixture is prepared by removal of para- and/or ortho-xylene from a fresh $C_8$ aromatic mixture obtained from an aromatics-production process, and preferably the non-equilibrium mixture contains less than 5 mass-% para-xylene.

The alkylaromatic hydrocarbons may be utilized in the present invention as found in appropriate fractions from various refinery petroleum streams, e.g., as individual components or as certain boiling-range fractions obtained by the selective fractionation and distillation of catalytically cracked or reformed hydrocarbons. The isomerizable aromatic hydrocarbons need not be concentrated; the process of this invention allows the isomerization of alkylaromatic-containing streams such as catalytic reformate with or without subsequent aromatics extraction to produce specified xylene isomers and particularly to produce para-xylene. A $C_8$-aromatics feed to the present process may contain nonaromatic hydrocarbons, i.e., naphthenes and paraffins, in an amount up to 30 mass-%. Preferably the isomerizable hydrocarbons consist essentially of aromatics, however, to ensure pure products from downstream recovery processes.

According to the process of the present invention, an alkylaromatic hydrocarbon feed mixture, preferably in admixture with hydrogen, is contacted with a catalyst of the type hereinafter described in an alkylaromatic hydrocarbon isomerization zone. Contacting may be effected using the catalyst in a fixed-bed system, a moving-bed system, a fluidized-bed system, or in a batch-type operation. In view of the danger of attrition loss of the valuable catalyst and of the simpler operation, it is preferred to use a fixed-bed system. In this system, a hydrogen-rich gas and the feed mixture are preheated by suitable heating means to the desired reaction temperature and then passed into an isomerization zone containing a fixed bed of catalyst. The conversion zone may be one or more separate reactors with suitable means therebetween to ensure that the desired isomerization temperature is maintained at the entrance to each zone. The reactants may be contacted with the catalyst bed in either upward-, downward-, or radial-flow fashion, and the reactants may be in the liquid phase, a mixed liquid-vapor phase, or a vapor phase when contacted with the catalyst.

The alkylaromatic feed mixture, preferably a non-equilibrium mixture of $C_8$ aromatics, is contacted with the isomerization catalyst at suitable alkylaromatic-isomerization conditions. Such conditions comprise a temperature ranging from 0° to 600°C or more, and preferably is in the range of from 100° to 500°C. The pressure generally is from 101 to 10132 kPa (1 to 100 atmospheres absolute) preferably less than 5066 kPa (50 atmospheres). Sufficient catalyst is contained in the isomerization zone to provide a liquid hourly space velocity with respect to the hydrocarbon feed mixture of from 0.1 to 30 $hr^{-1}$, and preferably 0.5 to 10 $hr^{-1}$. The hydrocarbon feed mixture optimally is reacted in admixture with hydrogen at a hydrogen/hydrocarbon mole ratio of 0.5:1 to 25:1 or more. Other inert diluents such as nitrogen, argon and light hydrocarbons may be present.

The reaction proceeds via the mechanism, described hereinabove, of isomerizing xylenes while reacting ethylbenzene to form a xylene mixture via conversion to and reconversion from naphthenes. The yield of xylenes in the product thus is enhanced by forming xylenes from ethylbenzene. The loss of $C_8$ aromatics through the reaction thus is low: typically less than 4 mass-% per pass of $C_8$ aromatics in the feed to the reactor, preferably 3 mass-% or less, and most preferably no more than 2.5 mass-%.

The particular scheme employed to recover an isomerized product from the effluent of the reactors of the isomerization zone is not deemed to be critical to the instant invention, and any effective recovery scheme known in the art may be used. Typically, the reactor effluent will be condensed and the hydrogen and light-hydrocarbon components removed therefrom by flash separation. The condensed liquid product then is fractionated to remove light and/or heavy byproducts and obtain the isomerized product. In some instances, certain product species such as ortho-xylene may be recovered from the isomerized product by selective fractionation. The product from isomerization of $C_8$ aromatics usually is processed to selectively recover the para-xylene isomer, optionally by crystallization. Selective adsorption is preferred using crystalline aluminosilicates according to US-A-3,201,491. Improvements and alternatives within the preferred adsorption recovery process are described in US-A-3,626,020, US-A-3,696,107, US-A-4,039,599, US-A-4,184,943, US-A-4,381,419 and US-A-4,402,832.

In a separation/isomerization process combination relating to the processing of an ethylbenzene/xylene mixture, a fresh $C_8$-aromatics feed is combined with isomerized product comprising $C_8$ aromatics and naphthenes from the isomerization reaction zone and fed to a para-xylene separation zone; the para-xylene-depleted stream comprising a non-equilibrium mixture of $C_8$ aromatics is fed to the isomerization reaction zone, where the $C_8$-aromatic isomers are isomerized to near-equilibrium levels to obtain the isomerized product. In this process scheme non-recovered $C_8$-aromatic isomers preferably are recycled to extinction until they are either converted to para-xylene or lost due to side-reactions. Ortho-xylene separation, preferably by fractionation, also may be effected on the fresh $C_8$-aromatic feed or isomerized product, or both in combination, prior to para-xylene separation.

The alkylation and preferably the monoalkylation of aromatic compounds involves reacting an aromatic compound with an olefin using the above described zeolitic catalyst. The olefins which can be used in the process are any

of those which contain from 2 up to 20 carbon atoms. These olefins may be branched or linear olefins and either terminal or internal olefins. Preferred olefins are ethylene, propylene and those olefins which are known as detergent range olefins. Detergent range olefins are linear olefins containing from 6 up through 20 carbon atoms which have either internal or terminal double bonds. Linear olefins containing from 8 to 16 carbon atoms are preferred and those containing from 10 up to 14 carbon atoms are especially preferred.

The alkylatable aromatic compounds may be selected from the group consisting of benzene, naphthalene, anthracene, phenanthrene, and substituted derivatives thereof, with benzene and its derivatives being the most preferred aromatic compound. By alkylatable is meant that the aromatic compound can be alkylated by an olefinic compound. The alkylatable aromatic compounds may have one or more of the substituents selected from the group consisting of alkyl groups (having from 1 to 20 carbon atoms), hydroxyl groups, and alkoxy groups whose alkyl group also contains from 1 up to 20 carbon atoms. Where the substituent is an alkyl or alkoxy group, a phenyl group can also can be substituted on the alkyl chain. Although unsubstituted and monosubstituted benzenes, naphthalenes, anthracenes, and phenanthrenes are most often used in the practice of this invention, polysubstituted aromatics also may be employed. Examples of suitable alkylatable aromatic compounds in addition to those cited above include biphenyl, toluene, xylene, ethylbenzene, propylbenzene, butylbenzene, pentylbenzene, hexylbenzene, heptylbenzene, octylbenzene, etc.; phenol, cresol, anisole, ethoxy-, propoxy-, butoxy-, pentoxy-, hexoxybenzene, etc.

The particular conditions under which the monoalkylation reaction is conducted depends upon the aromatic compound and the olefin used. One necessary condition is that the reaction be conducted under at least partial liquid phase conditions. Therefore, the reaction pressure is adjusted to maintain the olefin at least partially dissolved in the liquid phase. For higher olefins the reaction may be conducted at autogenous pressure. As a practical matter the pressure normally is in the range between 1379 and 6985 kPa (200-1000 psig) but usually is in a range between 2069-4137 kPa (300-600 psig). The alkylation of the alkylatable aromatic compounds with the olefins in the C2-C20 range can be carried out at a temperature of 60°C to 400°C, and preferably from 90°C to 250°C, for a time sufficient to form the desired product. In a continuous process this time can vary considerably, but is usually from 0.1 to 3 $hr^{-1}$weight hourly space velocity with respect to the olefin. In particular, the alkylation of benzene with ethylene can be carried out at temperatures of 200°C to 250°C and the alkylation of benzene by propylene at a temperature of 90°C to 200°C. The ratio of alkylatable aromatic compound to olefin used in the instant process will depend upon the degree of selective monoalkylation desired as well as the relative costs of the aromatic and olefinic components of the reaction mixture. For alkylation of benzene by propylene, benzene-to-olefin ratios may be as low as 1 and as high as 10, with a ratio of 2.5-8 being preferred. Where benzene is alkylated with ethylene a benzene-to-olefin ratio between 1:1 and 8:1 is preferred. For detergent range olefins of C6-C20, a benzene-to-olefin ratio of between 5:1 up to as high as 30:1 is generally sufficient to ensure the desired monoalkylation selectivity, with a range between 8:1 and 20:1 even more preferred.

The zeolites of this invention can also be used to catalyze transalkylation. By "transalkylation" is meant that process where an alkyl group on one aromatic nucleus is intermolecularly transferred to a second aromatic nucleus. A preferred transalkylation process is one where one or more alkyl groups of a polyalkylated aromatic compound is transferred to a nonalkylated aromatic compound, and is exemplified by reaction of diisopropylbenzene with benzene to give two molecules of cumene. Thus, transalkylation often is utilized to add to the selectivity of a desired selective monoalkylation by reacting the polyalkylates invariably formed during alkylation with nonalkylated aromatic to form additional monoalkylated products. For the purposes of this process, the polyalkylated aromatic compounds are those formed in the alkylation of alkylatable aromatic compounds with olefins as described above, and the nonalkylated aromatic compounds are benzene, naphthalene, anthracene, and phenanthrene. The reaction conditions for transalkylation are similar to those for alkylation, with temperatures being in the range of 100 to 250°, pressures in the range of 6985 to 3447 kPa (100 to 750 psig), and the molar ratio of unalkylated aromatic to polyalkylated aromatic in the range from 1 to 10. Examples of polyalkylated aromatics which may be reacted with, e.g., benzene as the nonalkylated aromatic include diethylbenzene, diisopropylbenzene, dibutylbenzene, triethylbenzene, triisopropylbenzene etc.

The X-ray patterns presented in the following examples (and tables above) were obtained using standard X-ray powder diffraction techniques. The radiation source was a high-intensity X-ray tube operated at 45 kV and 35 ma. The diffraction pattern from the copper K-alpha radiation was obtained by appropriate computer based techniques. Flat compressed powder samples were continuously scanned at 2° (2θ) per minute from 2° to 70°(2θ). Interplanar spacings (d) in Angstrom units were obtained from the position of the diffraction peaks expressed as 2θ where θ is the Bragg angle as observed from digitized data. Intensities were determined from the integrated area of diffraction peaks after subtracting background, "$I_o$" being the intensity of the strongest line or peak, and "I" being the intensity of each of the other peaks.

As will be understood by those skilled in the art, the determination of the parameter 20 is subject to both human and mechanical error, which in combination can impose an uncertainty of ±0.4 on each reported value of 2θ and up to ±0.5 on reported values for nanocrystalline materials. This uncertainty is, of course, also manifested in the reported values of the *d*-spacings, which are calculated from the θ values. This imprecision is general throughout the art and is not sufficient to preclude the differentiation of the present crystalline materials from each other and from the compositions

of the prior art. In some of the X-ray patterns reported, the relative intensities of the *d*-spacings are indicated by the notations vs, s, m and w which represent very strong, strong, medium, and weak, respectively. In terms of 100 X $I/I_o$, the above designations are defined as w = 0-15; m = 15-60; s = 60-80 and vs = 80-100. In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

In order to more fully illustrate the invention, the following examples are set forth. It is to be understood that the examples are only by way of illustration and are not intended as an undue limitation on the broad scope of the invention as set forth in the appended claims.

Example 1

An aluminosilicate reaction mixture was prepared in the following manner. Aluminum sec- butoxide (95+%), 58.75 g, was added to 836.34 g TEAOH (35%) with vigorous stirring. To this mixture, 294.73 g colloidal silica, (Ludox AS-40, 40% $SiO_2$) was added, followed by the addition of 10.18 g distilled water. The reaction mixture was homogenized for 1 hr with a high-speed mechanical stirrer, and then aged in teflon bottles overnight at 95°C. After the aging step, the reaction mixture was recombined and analyzed, the analysis indicated a silicon content of 4.67%.

A 500 g portion of this reaction mixture was treated with TMACl solution consisting of 11.77 g TMACl (97%) dissolved in 23.0 g distilled water while applying vigorous mixing. After a half-hour of homogenization the reaction mixture was distributed among 8 teflon-lined autoclaves. The autoclaves were all placed in ovens set at 150°C, where the reaction mixtures were digested for 4 days at autogenous pressures. The solid products were recovered by centrifugation, washed, and dried at 95°C.

The composition of the isolated products consisted of the mole ratios Si/Al = 6.88, N/Al = 0.83 and C/N = 6.05. Scanning Electron Microscopy (SEM) showed the crystallites to consist of clustered platelets approximately 100 - 300 nm across. Characterization by powder X-ray diffraction (XRD) showed the lines in the pattern to be those for the material designated UZM-5. Table 1 below shows lines characteristic of the phase. A portion of the sample was calcined by ramping to 540°C at 2°C/min in $N_2$, holding at 540°C in $N_2$ for 1 hr followed by a 7 hr dwell in air, also at 540°C. The BET surface area was found to be 530 $m^2/g$, and the micropore volume was 0.2cc/g.

Table 1

| 2-θ | d(Å) | $I/I_o$% |
|---|---|---|
| 6.24 | 14.15 | m |
| 7.90 | 11.18 | m |
| 10.32 | 8.57 | w-m |
| 12.00 | 7.37 | m |
| 15.80 | 5.60 | m-s |
| 16.34 | 5.42 | m |
| 19.05 | 4.66 | w-m |
| 22.00 | 4.04 | m |
| 22.86 | 3.89 | m |
| 23.80 | 3.74 | vs |
| 27.40 | 3.25 | w |
| 30.14 | 2.96 | w |
| 30.90 | 2.89 | w |
| 31.60 | 2.83 | m |
| 33.20 | 2.70 | w |
| 34.56 | 2.59 | w |
| 36.64 | 2.45 | w |
| 44.32 | 2.04 | w |

Example 2

An aluminosilicate reaction mixture was prepared in the following manner. Distilled water, 876.65 g was used to dilute 846.25 g TEAOH (35%) solution. Aluminum sec-butoxide (95+%), 49.54 g was added with vigorous stirring. This was followed by the addition of 427.57 g of TEOS (98%). The reaction mixture was heated to 85°C overnight and then distilled to 95°C for 2 hr to remove solvent. The reaction mixture was allowed to cool and was found to contain 3.34% Si by elemental analysis. A 300 g portion of this reaction mixture was placed in a teflon beaker and mixed vigorously. A solution containing 3.8 g TMACl (97%), 0.3 g LiCl, and 1.1 g $Sr(NO_3)_2$ dissolved in 25 g distilled water was prepared and added slowly to the aluminosilicate concoction with mixing. After the addition, the reaction mixture was homogenized for 2 hr and then split up among four teflon-lined autoclaves. The autoclaves were placed in a 150°C oven, where the reaction mixtures were digested for 5 days at 150°C at autogenous pressures. The products were recombined, the solids isolated by centrifugation, washed, and dried at 95°C.

Analysis by powder x-ray diffraction showed the product to have the UZM-5 structure. A portion of the product was calcined under a flow of nitrogen for 7 hr at 580°C. The composition of the calcined product exhibited the following mole ratios as determined by elemental analysis: Si/Al = 7.6, Sr/Al = 0.11, Li/Al = 0.06. The BET surface area of the calcined material was 500 $m^2/g$ and the micropore volume was 0.19cc/g. The SEM of the product showed the crystals to consist of a rosette morphology, the plate bundles usually on the order of 0.3 to 0.8 μ across. Characteristic lines in the x-ray diffraction pattern are shown below in Table 2.

Table 2

| 2-θ | d(Å) | $I/I_o$% |
|---|---|---|
| 6.12 | 14.43 | m |
| 7.84 | 11.27 | m |
| 10.18 | 8.68 | w-m |
| 11.90 | 7.43 | m |
| 15.78 | 5.61 | m-s |
| 19.04 | 4.66 | w-m |
| 20.38 | 4.35 | w |
| 21.90 | 4.05 | m |
| 22.80 | 3.90 | m |
| 23.70 | 3.75 | vs |
| 25.22 | 3.53 | w |
| 26.26 | 3.39 | w |
| 30.06 | 2.97 | w |
| 31.40 | 2.85 | w-m |
| 33.18 | 2.70 | w |
| 44.13 | 2.05 | w |

Example 3

An aluminosilicate reaction mixture was prepared by adding 17.22 g of $Al(Osec\text{-}Bu)_3$ (95+%) to 470.69 g TEAOH (35%) with vigorous stirring. De-ionized water was added, 8.41 g, followed by the addition of 103.67 g of Ludox AS-40 colloidal silica. The reaction mixture was homogenized for an hour with a high-speed stirrer before it was placed in a teflon bottle and aged at 95°C for 2 days. After it was cooled it was determined by elemental analysis that the reaction mixture contained 3.46% Si. A 50 g portion of this reaction mixture was treated with a solution of TMACl (97%), 0.71 g, dissolved in 10 g de-ionized water. The mixture was homogenized for 30 minutes with a high-speed stirrer and then split among 3 teflon-lined autoclaves and digested for 2, 4, and 6 days at 150°C at autogenous pressures. The solid products were isolated by centrifugation, washed with distilled water, and dried at 95°C.

The products of the reaction mixtures digested for 4 and 6 days were shown to be UZM-5 by powder x-ray diffraction. Characteristic lines in the x-ray diffraction pattern for the material observed after 6 days are given in Table 3.

Table 3

| 2-θ | d(Å) | $I/I_o$% |
|---|---|---|
| 5.90 | 14.97 | m |
| 7.66 | 11.53 | m |
| 10.06 | 8.78 | w-m |
| 11.70 | 7.56 | m |
| 15.70 | 5.64 | m-s |
| 16.36 | 5.41 | w-m |
| 18.74 | 4.73 | w-m |
| 20.36 | 4.36 | w |
| 21.84 | 4.07 | m |
| 22.72 | 3.91 | w-m |
| 23.56 | 3.77 | vs |
| 26.20 | 3.40 | w |
| 27.26 | 3.27 | w |
| 29.94 | 2.98 | w |
| 31.26 | 2.86 | w-m |
| 33.08 | 2.71 | w |
| 43.92 | 2.06 | w |

Example 4

This example shows the substitution of gallium for some of the aluminum in the structure. Two separate reaction mixtures, Mixture A, an aluminosilicate composition, and Mixture B, a gallosilicate composition, were prepared. Mixture A was prepared by adding 116.06 g $Al(OsecBu)_3$ (95+%) to 1982.41 g TEAOH (35%) with vigorous stirring. De-ionized water, 2.92 g, was added to the stirring reaction mixture along with 698.62 g Ludox AS-40 colloidal silica. The resulting mixture was homogenized for an hour with a high-speed stirrer before it was placed in teflon bottles and aged at 95°C for one day. This aluminosilicate composition, Mixture A, contained 4.96% Si by analysis and a Si/Al ratio of 9.53. Mixture B was prepared by diluting 275.23 g TEAOH (35%) with 275.23 g de-ionized water. To this vigorously stirring solution, 107.77 g Ludox AS-40 colloidal silica and 36.2 g freshly precipitated $Ga(OH)_3$•$xH_2O$, 13.8% Ga, were added. After an hour of homogenization, the reaction mixture was placed in a teflon bottle and aged at 95°C for three days. This gallosilicate composition, Mixture B, contained 3.21 % Si by analysis.

The substituted aluminosilicate was prepared by combining 45 g Mixture A with 5 g Mixture B in a teflon beaker while employing high-speed stirring. A solution prepared by dissolving 1.17 g TMACl (97%) in 10.0 g de-ionized water was added to the stirring mixture. After a half-hour of homogenization, the reaction mixture was distributed among three autoclaves and digested for 4, 6, and 8 days at 150°C at autogenous pressures. The solid products were isolated by centrifugation, washed with de-ionized water, and dried at 95°C.

All three of the products were shown to have the UZM-5 structure by powder x-ray diffraction. Elemental analysis showed the six day sample to consist of the following mole ratios: Si/(Al+Ga) = 7.35; N/(Al+Ga) = 1.11; Si/Ga = 78.3; Al/Ga = 15.0 and C/N = 5.44. Characteristic lines in the x-ray diffraction pattern are given in table 4.

Table 4

| 2-θ | d(Å) | $I/I_o$% |
|---|---|---|
| 6.06 | 14.57 | m |
| 7.80 | 11.33 | m |
| 10.16 | 8.70 | w-m |

(continued)

| 2-θ | d(Å) | $I/I_o$% |
|---|---|---|
| 11.84 | 7.46 | m |
| 15.72 | 5.63 | m-s |
| 19.04 | 4.66 | w-m |
| 20.38 | 4.35 | w |
| 21.822 | 4.07 | m |
| 22.76 | 3.90 | w-m |
| 23.66 | 3.76 | vs |
| 26.22 | 3.40 | w |
| 30.06 | 2.97 | w |
| 31.44 | 2.84 | w-m |
| 33.14 | 2.70 | w-m |
| 34.50 | 2.60 | w |
| 44.04 | 2.05 | w |

Example 5

An aluminosilicate reaction mixture was prepared by adding 197.31 g $Al(Osec\text{-}Bu)_3$ to 2808.74 g TEAOH (35%), followed by the addition of 989.82 g colloidal silica (Ludox AS-40) while maintaining vigorous stirring. The reaction mixture was aged at 95°C for 16 hr and then allowed to cool. This aluminosilicate reaction mixture was designated Mixture C and was used again in Example 7. Elemental analysis showed Mixture C to contain 4.79% Si. A portion of this reaction mixture, 110 g, was placed in a teflon beaker equipped with a high-speed stirrer. Separately, a solution was prepared by dissolving 1.27 g TMACl (97%) and 0.68 g NaCl in 6 g de-ionized water. This solution was added to the stirring aluminosilicate reaction mixture. After a half-hour of homogenization, the reaction mixture was divided among 4 teflon-lined autoclaves which were digested under a variety of conditions. The solid products were isolated by filtration, washed with de-ionized water, and dried at 95°C.

The products of all of the reactions exhibited the x-ray diffraction pattern of UZM-5. Characteristic lines in the x-ray diffraction pattern of a sample digested at 150°C for 4 days are shown in table 5. Scanning Electron Microscopy showed the sample to be very uniform, consisting of rosettes of small plate-like crystals with the rosettes measuring from 0.5 to 2 μ across. The BET surface area for this material was found to be 553 $m^2/g$, while the micropore volume was 0.22 cc/g. Elemental analysis showed the Si/Al ratio to be 5.97, Na/Al = 0.19, N/Al = 0.97, and C/N = 5.59. Characteristic lines in the x-ray diffraction pattern for this material are given in table 5.

Table 5

| 2-θ | d(Å) | $I/I_o$% |
|---|---|---|
| 6.16 | 14.33 | m |
| 7.76 | 11.39 | s |
| 10.12 | 8.73 | m |
| 11.82 | 7.48 | m |
| 15.74 | 5.63 | vs |
| 19.04 | 4.66 | m |
| 20.36 | 4.36 | w |
| 21.84 | 4.07 | m |
| 22.68 | 3.92 | s |
| 23.56 | 3.77 | vs |

(continued)

| 2-θ | d(Å) | $I/I_o$% |
|---|---|---|
| 26.18 | 3.40 | w |
| 27.02 | 3.30 | m |
| 29.98 | 2.98 | m |
| 31.32 | 2.85 | m |
| 33.12 | 2.70 | m |
| 44.10 | 2.05 | w |

Example 6

An aluminosilicate reaction mixture was prepared using the following procedure: Aluminum sec-butoxide (95+%), 987.54 g, was added to 14058 g TEAOH (35%) with vigorous stirring. This was followed by the addition of 4954 g colloidal silica, Ludox AS-40. The reaction mixture was aged with stirring at 95°C for 16 hr. After aging, the reaction mixture was found to contain 4.72% Si. This aluminosilicate reaction mixture was identified as Mixture D, and was used again in example 9. A portion of Mixture D, 47.01 g, was placed in a beaker equipped with a stirrer. Separately, a solution was prepared by dissolving 1.12 g TMACl (97%) in 1.87 g de-ionized water. While stirring the aluminosilicate reaction mixture, the TMACl solution was added, and the resulting mixture was further homogenized for 20 minutes. The reaction mixture was then placed in a 100 ml Parr stirred autoclave. The temperature of the reaction mixture was ramped from room temperature to 150°C over a period of 3 hr, before it was held at 150°C for 24 hr. The reaction mixture was digested at autogenous pressures. The solid reaction product was isolated by centrifugation, washed with de-ionized water, and dried at 100°C.

The product exhibited the diffraction pattern of UZM-5P. The characteristic x-ray diffraction lines for this material are shown in table 6 below. A reaction mixture of the same formulation, but digested for 72 hr instead of 24 hr, yielded well-crystallized UZM-5. The morphology of UZM-5P was plate-like with sub-micron dimensions as determined by Scanning Electron Microscopy. Elemental analysis showed the Si/Al ratio to be 6.0, N/Al = 1.00 and C/N = 6.07.

Table 6

| 2-θ | d(Å) | $I/I_o$% |
|---|---|---|
| 6.16 | 14.33 | vs |
| 7.62 | 11.60 | m |
| 10.26 | 8.61 | s |
| 16.23 | 5.46 | m |
| 19.06 | 4.65 | m |
| 22.86 | 3.89 | vs |
| 30.38 | 2.94 | m |
| 44.22 | 2.05 | w |

Example 7

A UZM-5P composition similar to that disclosed in example 6 was prepared in the following manner: 40 g of Mixture C (see Example 5), was used as the aluminum and silicon source and was placed in a beaker equipped with a high-speed stirrer. Separately, 0.92 g TMACl (97%) was dissolved in 40 g de-ionized water. This solution was added to Mixture C with vigorous stirring. After 20 minutes of agitation the reaction mixture was placed in a teflon-lined autoclave and digested at 150°C for 4 days at autogenous pressures. The solid products were isolated by centrifugation, washed with de-ionized water, and dried at 95°C.

The product exhibited an x-ray diffraction pattern indicative of UZM-5P. Characteristic x-ray diffraction lines for this material are given in Table 7. Elemental analysis showed the material to have Si/Al = 6.03, N/Al = 1.07, and C/N = 6.11. Scanning Electron Microscopy showed the sample to consist of clusters of bent plate-like crystals forming rosettes 400 - 800 nm across.

Table 7

| 2-θ | d(Å) | $I/I_o$% |
|---|---|---|
| 5.80 | 15.23 | s |
| 7.77 | 11.37 | m |
| 10.22 | 8.65 | m |
| 11.66 | 7.58 | m |
| 16.15 | 5.48 | m |
| 21.93 | 4.05 | w |
| 22.84 | 3.89 | m |
| 23.54 | 3.78 | vs |
| 30.00 | 2.98 | w |
| 33.02 | 2.71 | w |
| 44.04 | 2.05 | w |

Example 8

An aluminosilicate reaction mixture was prepared in the following manner: TEAOH (35%), 846.25 g, was diluted with 876.65 g de-ionized water. $Al(O\text{-}secBu)_3$ (95+%), 49.54 g, was added to the hydroxide solution followed by the addition of TEOS (98%), 427.57 g, while maintaining vigorous stirring. After two hours of homogenization, the reaction mixture was placed in a flask and aged at 75°C with light stirring overnight. After the aging, the flask was fitted with a distillation head and some of the alcoholic hydrolysis products were removed via distillation. After solvent removal, the reaction mixture contained 3.34% Si. A 1200 g portion of this aluminosilicate reaction mixture was placed in a beaker equipped with a high-speed mixer. Separately, a solution was prepared by dissolving 15 g TMACl (97%) in 30 g de-ionized water. This solution was added dropwise to the aluminosilicate reaction mixture and further homogenized for a half -hour. The reaction mixture was then placed in a Parr 2-L static autoclave equipped with a teflon-cup liner. The reaction mixture was digested at 150°C for 90 hr at autogenous pressures. The solid product was isolated by centrifugation, washed with de-ionized water, and dried at 95°C.

The product had an x-ray diffraction pattern consistent with that of the UZM-5P material, most notably the peaks at d=8.68Å and 3.89Å. Elemental analysis showed the Si/Al ratio = 10.05, while the BET surface area was 601 $m^2/g$ and the micropore volume 0.21 cc/g. Scanning Electron Microscopy showed the material to consist of uniform clusters of plate-like crystals in a rosette formation, with the rosettes having a diameter of 0.25 to 0.5 μ. Characteristic lines in the x-ray pattern for this material are given in Table 8 below.

Table 8

| 2-θ | d(Å) | $I/I_o$% |
|---|---|---|
| 7.58 | 11.65 | m |
| 10.18 | 8.68 | m |
| 16.36 | 5.41 | m |
| 22.84 | 3.89 | m |
| 23.60 | 3.77 | vs |

Example 9

A series of zeolites were prepared in a similar manner as the zeolite in example 6. In a beaker there were placed 47.01 g of mixture D (see Example 6) and to it there was added with stirring a solution of 1.12g TMACl (97%) in 1.87g of de-ionized water. The resulting mixture was homogenized for 20 minutes and then transferred to a 100ml Parr stirred autoclave. Four other mixtures were prepared in a similar manner and the respective mixtures were heated to 150°C and held at 150°C for 12 hrs, 18 hrs, 24 hrs, 36 hrs, and 72 hrs under autogenous pressure. Each solid reaction

product was isolated by centrifugation, washed with de-ionized water and then dried at 100°C.

The x-ray diffraction patterns of the five samples are presented in Table 9. The data show that additional diffraction lines are observed as the amount of digestion time is increased. It is clear that the peaks at d ≈ 8.6Å and d = 3.9Å (in bold) are common to all the synthesized materials, i.e. structures.

Without wishing to be bound by any particular theory, the following explanation is proposed. UZM-5 can be indexed on a tetragonal cell with a = 12.4 Å and c = 28.6 Å. Based on a tetragonal cells the 8.6Å and 3.9Å peaks have indices of 110 and 310 respectively. This suggest that ordering first occurs in the *a and b* directions and then in the c-direction. It appears that a large number of structures can be prepared by stopping the reaction at various times until 36 hours where the UZM-5 structure is attained. By stopping the synthesis at various times, one can obtain zeolites with a range of different surface area, acidity and adsorption properties.

Table 9

| **12 hr UZM-5P** | | **18 hr UZM-5P** | | **24 hr UZM-5P** | | **36 hr UZM-5** | | **72 hr UZM-5** | |
|---|---|---|---|---|---|---|---|---|---|
| d(Å) | I% | d(Å) | I% | d(Å) | I% | d(Å) | I% | d(Å) | I% |
| | | 16.00 | 100 | 14.33 | 100 | 14.92 | 47 | 14.29 | 37 |
| | | | | 11.27 | 19 | 11.38 | 28 | 11.33 | 33 |
| ***8.57*** | ***38*** | ***8.68*** | ***32*** | ***8.61*** | ***62*** | ***8.73*** | ***25*** | ***8.72*** | ***24*** |
| | | | | | | 7.43 | 22 | 7.44 | 33 |
| | | 5.40 | 9 | 5.53 | 39 | 5.62 | 60 | 5.66 | 61 |
| | | | | | | | | 5.51 | 42 |
| | | | | 4.65 | 17 | 4.66 | 17 | 4.68 | 22 |
| | | | | | | | | 4.48 | 2 |
| | | | | | | 4.33 | 2 | 4.35 | 5 |
| | | | | | | 4.06 | 5 | 4.06 | 51 |
| | | | | | | | | 3.96 | 49 |
| ***3.88*** | ***100*** | ***3.88*** | ***50*** | ***3.89*** | ***88*** | ***3.90*** | ***14*** | ***3.90*** | ***25*** |
| | | | | 3.75 | 54 | 3.76 | 100 | 3.76 | 100 |
| | | | | | | | | 3.55 | 6 |
| | | | | | | 3.39 | 6 | 3.40 | 6 |
| | | | | | | 3.28 | 8 | 3.29 | 12 |
| | | 2.93 | 12 | 2.95 | 16 | 2.98 | 10 | 2.97 | 11 |
| | | | | 2.84 | 8 | 2.85 | 15 | 2.85 | 17 |
| | | | | | | 2.70 | 13 | 2.71 | 13 |
| | | | | | | | | 2.60 | 7 |
| | | 2.06 | 5 | 2.05 | 14 | 2.05 | 8 | 2.05 | 6 |

Example 10

An aluminosilicate reaction mixture was prepared in the following manner: $Al(Osec\text{-}Bu)_3$ (95+%), 116.09 g, was added to 1983.17 g TEAOH (35%) and 1.86g de-ionized water with vigorous stirring. Then 698.88 g Ludox AS-40 was added, with continued stirring. After an hour of homogenization, the aluminosilicate reaction mixture was placed in several teflon bottles and aged at 95°C for 3 days. After the aging process, elemental analysis showed the mixture to contain 5.01% Si and had a Si/Al ratio of 10.03. This reaction mixture is designated Mixture E. A portion of this aluminosilicate reaction mixture, 40.0 g, was placed in a beaker where it was stirred vigorously. Separately, 0.78 g TMACl (97%) was dissolved in 15.0 g de-ionized water. This solution was added to the stirring aluminosilicate reaction mixture in a dropwise fashion. The mixture was allowed to homogenize further for an hour. The reaction mixture was then placed

in a teflon-lined autoclave and digested at 150°C for 6 days at autogenous pressures. The solid product was isolated by centrifugation, washed with de-ionized water, and dried at 95°C.

The product had an x-ray pattern consistent with UZM-6. Scanning Electron Microscopy (SEM) showed the material to consist of plate-like crystals 0.1 - 0.4 μ across and less than 0.05 μ thick. The Si/Al ratio of the product UZM-6 was 8.34 by elemental analysis. The BET surface area of the sample was 520 $m^2$/g, with a micropore volume of 0.21 cc/g. Characteristic lines in the x-ray diffraction pattern are given in table 10.

Table 10

| 2-θ | d(Å) | $I/I_o$% |
|---|---|---|
| 6.14 | 14.38 | m |
| 7.76 | 11.38 | m |
| 10.12 | 8.73 | m |
| 11.82 | 7.48 | m |
| 15.68 | 5.65 | s |
| 16.30 | 5.43 | m |
| 18.98 | 4.67 | m |
| 20.32 | 4.37 | w |
| 21.86 | 4.06 | m |
| 22.42 | 3.96 | s |
| 22.78 | 3.90 | m |
| 23.68 | 3.75 | vs |
| 25.24 | 3.53 | w |
| 26.28 | 3.39 | w |
| 26.88 | 3.31 | m |
| 27.34 | 3.26 | m |
| 29.64 | 3.01 | m |
| 30.08 | 2.97 | w |
| 31.44 | 2.84 | w |
| 33.20 | 2.70 | w |
| 44.14 | 2.05 | w |

Example 11

An aluminosilicate reaction mixture was prepared in an identical manner to Mixture E described in example 10. However, the reaction mixture was determined to be slightly different by analysis, with a Si content of 4.79 wt % and a Si/Al ratio of 9.59. A portion of this aluminosilicate reaction mixture, 1100 g, was placed in a large beaker equipped with a high-speed stirrer. Separately, a solution was prepared by dissolving 4.14 g LiCl and 21.43 g TMACl (97%) in 65 g de-ionized water. This solution was added dropwise to the aluminosilicate reaction mixture with stirring and was homogenized for an hour. The reaction mixture was then transferred to a static 2-L Parr reactor and digested at 150°C for 3 days at autogenous pressure. The solid product was isolated by filtration, washed with de-ionized water and dried at 95°C.

Powder x-ray diffraction on a sample of the product showed the pattern to be consistent with that for UZM-6. The Si/Al ratio was 7.58. The BET surface area was 512 $m^2$/g, while the micropore volume was found to be 0.18 cc/g. SEM of the calcined product showed it to consist of bent plate crystals, sometimes stacked, up to 0.1 - 0.4 μ across and less that 0.05 μ thick. Characteristic lines in the x-ray diffraction pattern are given in table 11.

Table 11

| 2-θ | d(Å) | $I/I_o$% |
|---|---|---|
| 6.28 | 14.07 | m |
| 7.84 | 11.27 | s |
| 10.22 | 8.65 | m |
| 11.92 | 7.42 | m |
| 15.93 | 5.56 | m |
| 18.98 | 4.67 | m |
| 21.98 | 4.04 | w |
| 22.52 | 3.95 | vs |
| 22.92 | 3.88 | m |
| 23.76 | 3.74 | vs |
| 26.33 | 3.38 | w |
| 26.92 | 3.31 | m |
| 31.36 | 2.85 | m |
| 33.26 | 2.69 | m |
| 44.24 | 2.05 | w |

Example 12

It is known that zeolites are capable of a variety of hydrocarbon conversion processes and find much of their utility in this regard. This example demonstrates the capability of UZM-5, UZM-5P, and UZM-6 to convert heptane to a variety of products. Calcined samples of these materials and for comparison, a steam-stabilized‘ Y zeolite (SSY) were tested in a microreactor operating at atmospheric pressure. The UZM-5 from example 5 and the UZM-6 from example 11 were ammonium ion-exchanged after calcination to remove alkali and obtain the acid form. The feed was heptane, saturated at 0°C in an $H_2$ carrier gas. The catalyst loading was 250 mg of 40-60 mesh particulates. The samples were pretreated at 550°C for 60 minutes in $H_2$. The feed was introduced at a constant flow rate of 125 cc/min. The products were hydrogenated before going to the gas chromatograph. In this variable temperature program, the product stream was sampled at the following temperatures/times on stream: 25°C/0 hr, 450°C/0.33 hr, 500°C/ 1.10 hr and 1.45 hr, and 550°C/2.20 hr and 2.55 hr. The selectivities to the major products for each sample are given in Table 12 for the last data point collected at 550°C. The data show that UZM-5 and UZM-5P are comparable to SSY in ability to convert heptane, while UZM-6 is clearly much more active than SSY in heptane conversion. UZM-6 shows much more conversion to aromatic products than the other materials.

Table 12

| Sample | SSY | Ex.1 UZM-5 | Ex. 5 UZM-5 | Ex.8 UZM-5P | Ex.11 UZM-6 |
|---|---|---|---|---|---|
| Temperature | 550°C | 550°C | 550°C | 550°C | 550°C |
| Time on Stream (hr) | 2.55 | 2.55 | 2.55 | 2.55 | 2.55 |
| Methane | 0.58 | 1.35 | 1.67 | 0.44 | 3.27 |
| Ethane | 3.21 | 3.90 | 5.33 | 1.78 | 10.22 |
| Propane | 17.14 | 17.15 | 22.98 | 8.69 | 30.88 |
| Isobutane | 9.9 | 5.12 | 7.04 | 4.61 | 14.23 |
| n-Butane | 8.61 | 8.40 | 10.25 | 5.13 | 11.35 |
| Isopentane | 1.72 | 1.16 | 1.43 | 1.37 | 1.52 |

(continued)

| Sample | SSY | Ex.1 UZM-5 | Ex. 5 UZM-5 | Ex.8 UZM-5P | Ex.11 UZM-6 |
|---|---|---|---|---|---|
| Temperature | 550°C | 550°C | 550°C | 550°C | 550°C |
| n-Pentane | 1.18 | 1.24 | 1.45 | 0.65 | 1.26 |
| Benzene | 0.19 | 0.12 | 0.29 | 0.17 | 1.66 |
| Heptane | 55.58 | 60.73 | 48.31 | 75.98 | 19.9 |
| Toluene | 1.75 | 0.46 | 0.88 | 0.75 | 4.19 |

Example 13

Another distinguishing feature of zeolites is the ability to adsorb molecules in their micropores. Such properties enable the utility of zeolites in adsorbent, separation, and selective catalytic applications. Adsorption measurements were performed using a standard McBain-Bakr gravimetric adsorption apparatus. The samples were calcined at 560°C to remove organic templates before they were loaded into the tubes. The samples were then activated at 350°C overnight to remove adsorbed water from the pores. The samples were then exposed to a gas at several specific partial pressures and the amount of the gas adsorbed is expressed in terms of the weight percent of the sample. The data in the tables below demonstrate the ability of UZM-5 and UZM-5P to adsorb n-butane, isobutane, $O_2$, and $SF_6$

| Adsorption of $O_2$ (T = -183°C) | | | |
|---|---|---|---|
| $P/P_o$ (Partial pressure) | Ex.1 UZM-5 wt % $O_2$ | Ex. 5 UZM-5 Wt % $O_2$ | Ex. 8 UZM-5P wt % $O_2$ |
| 0.13 | 24.8 | 26.1 | 17.4 |
| 0.39 | 28.3 | 28.7 | 25.4 |
| 0.92 | 37.1 | 34.2 | 39.2 |

| Adsorption of n-butane T = 22°C | | | |
|---|---|---|---|
| $P/P_o$ (Partial pressure) | Ex. 1 UZM-5 wt % n-$C_4H_{10}$ | Ex. 5 UZM-5 wt % n-$C_4H_{10}$ | Ex. 8 UZM-5P wt % n-$C_4H_{10}$ |
| 0.04 | 7.9 | 9.4 | 4.6 |
| 0.12 | 9.6 | 11.0 | 7,1 |
| 0.30 | 11.7 | 12.7 | 11.4 |

| Adsorption of isobutane T = 22°C | | | |
|---|---|---|---|
| $P/P_o$ (Partial pressure) | Ex. 1 UZM-5 wt % i-$C_4H_{10}$ | Ex. 5 UZM-5 wt % i-$C_4H_{10}$ | Ex. 8 UZM-5P wt % i-$C_4H_{10}$ |
| 0.04 | 1.7 | 1.5 | 3.1 |
| 0.12 | 2.6 | 2.0 | 5.7 |
| 0.30 | 4.1 | 2.8 | 9.3 |

| Adsorption of $SF_6$ T = 22°C | | | |
|---|---|---|---|
| $P/P_o$ (Partial pressure) | Ex. 1 UZM-5 wt % $SF_6$ | Ex. 5 UZM-5 wt % $SF_6$ | Ex. 8 UZM-5P wt % $SF_6$ |
| 0.92 | 3.3 | 1.8 | 5.6 |

Example 14

Another method for examining adsorption properties is to use TGA or Thermal Gravimetric Analysis. In such an apparatus, adsorption information at elevated temperatures is easy to acquire, as well as controlled delivery of the adsorption gasses. This is especially convenient for larger, less volatile adsorbates that are more easily studied and handled at higher temperatures. The tests were conducted on calcined samples. Approximately 50 mg of sample was placed in the TGA sample holder. The sample was then activated for 2 hr at 500°C in a $N_2$ stream flowing at 127 cc/min. After the pretreatment, the sample was brought to 120°C and an additional feed was cut in consisting of $N_2$ saturated with cis 1,2-dimethylcyclohexane at 25°C, which flows at 72 cc/min. Hence, the total flow rate past the sample was 200 cc/min. The adsorption of the cis 1,2-dimethylcyclohexane was then monitored by TGA at 120°C for an additional 250 minutes. The cis 1,2-dimethylcyclohexane adsorption after 5 and 250 minutes are given in terms of wt.% of the sample in table 14. The table shows that UZM-6 picks up the large cis 1,2-dimethylcyclohexane much more readily than UZM-5.

Table 14

| cis 1,2-dimethylcyclohexane (DMC) adsorption, T = 120°C | | |
|---|---|---|
| | Ex. 1, UZM-5 wt. % cis 1,2-DMC | Ex. 10, UZM-6 wt. % cis 1,2-DMC |
| 5 minutes | 0.59 | 2.08 |
| 250 minutes | 1.18 | 2.59 |

Example 15

Samples from examples 1 to 3 were tested for alkylation activity by using an ethylbenzene disproportionation test. The materials were converted to the proton form before testing. The UZM-6 from example 3 was calcined in air at 350°C for 1.5 hours, 450°C for 1.5 hours and 7 hours at 580°C and ion exchanged with ammonium chloride, three times at 80°C for 2 hours The UZM-6 from example 2 was calcined at 520°C for 1 hour in $N_2$, followed by 19 hours in air. The UZM-5 from example 1 was calcined at 520°C for 10 hr. The calcined samples were sized to 40 - 60 mesh and loaded (250 mg) into a quartz tube (11 mm i.d.) reactor residing in a furnace. The outlet pressure at the reactor inlet was atmospheric pressure. The samples were pretreated at 250°C in a flow of $N_2$. The temperature was brought down to 150°C and then the feed was introduced. The feed consisted of the $N_2$ flow passing through an ethylbenzene saturator held at 0°C, with the $N_2$ flow controlled at 150 cc/min. While the flow remained constant, the sample was exposed to the feed and reaction products were examined at 150°C, 150°C, 125°C, 175°C, 200°C, 230°C, and 175°C. The product effluents are analyzed by an on-line GC to measure activity and selectivity. Results from the second 150°C and the 230°C product collections are given in Table 15.

Table 15

| | **UZM-5, Ex. 1** | | **UZM-6, Ex. 2** | | **UZM-6, Ex. 3** | |
|---|---|---|---|---|---|---|
| Temperature (deg C) | 150 | 230 | 150 | 230 | 150 | 230 |
| Flow Rate (cc/min) | 150 | 150 | 150 | 150 | 150 | 150 |
| | | | | | | |
| Methane/Ethane | 0.02 | 0 | 0.03 | 0.06 | 0 | 0.08 |
| C4's | 0 | 0 | 0.02 | 0.07 | 0.1 | 0.11 |
| Benzene | 0.09 | 0.18 | 0.39 | 1.21 | 0.64 | 2.63 |

(continued)

| | UZM-5, Ex. 1 | | UZM-6, Ex. 2 | | UZM-6, Ex. 3 | |
|---|---|---|---|---|---|---|
| Ethylcyclohexane | 0 | 0 | 0 | 0 | 0 | 0 |
| Toluene | 0 | 0 | 0.04 | 0.04 | 0.04 | 0.05 |
| Ethylbenzene | 99.72 | 99.46 | 98.69 | 96.02 | 97.86 | 91.97 |
| p-Xylene | 0 | 0 | 0 | 0 | 0 | 0 |
| m-Xylene | 0 | 0 | 0 | 0 | 0 | 0 |
| o-Xylene | 0 | 0 | 0 | 0 | 0 | 0 |
| 1 E4MBZ | 0 | 0 | 0 | 0 | 0 | 0 |
| 1 E3MBZ | 0 | 0 | 0 | 0 | 0 | 0 |
| 1 E2MBZ | 0 | 0 | 0 | 0 | 0 | 0 |
| 13DEBZ | 0.09 | 0.22 | 0.41 | 1.63 | 0.76 | 3.24 |
| 14DEBZ | 0.08 | 0.13 | 0.4 | 0.82 | 0.55 | 1.6 |
| 12DEBZ | 0 | 0 | 0.03 | 0.15 | 0.05 | 0.28 |
| 14DIPBZ | 0 | 0 | 0 | 0 | 0 | 0.02 |
| 135TEBZ | 0 | 0 | 0 | 0 | 0 | 0 |
| 124TEBZ | 0 | 0 | 0 | 0 | 0 | 0 |
| | | | | | | |
| EB CONV | 0.28 | 0.54 | 1.31 | 3.98 | 2.14 | 8.03 |

Example 16

This example demonstrates the capability of UZM-6 to catalyze the synthesis of ethylbenzene from benzene and ethylene. The zeolite from example 3 was converted to the proton by the same procedure as in example 15. The zeolite was then bound with Plural SB alumina in a 70% zeolite/30% alumina formulation and formed into 1/16" diameter extrudates. The extrudates were then calcined at 550°C for 2 hr. The test employs a 7/8" diameter stainless steel reactor which is loaded with 40 cc of the catalyst. The benzene and ethylene are mixed on-line to a 3/1 benzene/ethylene ratio and then pre-heated before entering the reactor. The olefin was added at a rate of 0.45 $hr^{-1}$ LHSV. The testing was done with an effluent recycle to control the free olefin at the reactor inlet. The reaction was carried out at 500 psig. Activity and selectivity data were collected at 200°C and 230°C. The selectivities to alkylated products are shown in Table 16.

Table 16

| Ethylbenzene Synthesis with UZM-6 | | |
|---|---|---|
| **Hours on Stream** | 60 | 271 |
| **Inlet temperature** | 200°C | 230°C |
| **Ethylbenzene** | 80.31 | 74.95 |
| **Diethylbenzenes** | 16.26 | 19.95 |
| **Triethylbenzenes** | 2.46 | 3.92 |
| **Tetraethylbenzenes** | 0.22 | 0.37 |
| **Alkylated products** | 99.25 | 99.19 |

Example 17

An aluminosilicate reaction mixture was prepared in an identical manner to Mixture B described in example 3. However, the reaction mixture was determined to be slightly different by analysis, with a Si content of 4.79 wt % and a

Si/Al ratio of 9.59. A portion of this aluminosilicate reaction mixture, 1100 g, was placed in a large beaker equipped with a high-speed stirrer. Separately, a solution was prepared by dissolving 4.14 g LiCl and 21.43 g TMACl (97%) in 65 g de-ionized water. This solution was added dropwise to the aluminosilicate reaction mixture with stirring and was homogenized for an hour. The reaction mixture was then transferred to a static 2-L Parr reactor and digested at 150°C for 3 days at autogenous pressure. The solid product was isolated by filtration, washed with de-ionized water and dried at 95°C.

Powder x-ray diffraction on a sample of the product showed the pattern to be consistent with that known for UZM-6. The Si/Al ratio was 7.58. The BET surface area was 512 $m^2/g$, while the micropore volume was found to be 0.18 cc/g. SEM of the calcined product showed it to consist of bent plate crystals, sometimes stacked, up to 0.1 - 0.4 μ across and less that 0.05 μ thick. Characteristic lines in the x-ray diffraction pattern are given in Table 17.

Table 17

| 2-θ | d(Å) | $I/I_o$% |
|---|---|---|
| 6.28 | 14.07 | m |
| 7.84 | 11.27 | s |
| 10.22 | 8.65 | m |
| 11.92 | 7.42 | m |
| 15.93 | 5.56 | m |
| 18.98 | 4.67 | m |
| 21.98 | 4.04 | w |
| 22.52 | 3.95 | vs |
| 22.92 | 3.88 | m |
| 23.76 | 3.74 | vs |
| 26.33 | 3.38 | w |
| 26.92 | 3.31 | m |
| 31.36 | 2.85 | m |
| 33.26 | 2.69 | m |
| 44.24 | 2.05 | w |

Example 18

The zeolites from examples 1 to 3 and 17 were tested for xylene isomerization. The materials were converted to an acid form before testing. Material isolated from the procedure of example 1 was tested after calcination at 550°C in air for 5 hr. This sample was designated UZM-5, Ex.1. A portion of the same material was acid washed (9g sample, 2 g $H_2SO_4$ (98%) in 60 g de-ionized water, 90°C, 2 hr), washed with water, and ammonium ion exchanged (1 N $NH_4Cl$, 90°C, 1.5 hr) before calcination at 550°C for 5 hr. This sample was designated UZM-5, Ex.1-AW. The material from example 2 was calcined at 550°C for 5 hr, ammonium ion exchanged 3 times (1 N $NH_4Cl$, 75°C), and calcined at 550°C for 2 hr. This sample was designated UZM-5, Ex. 2. The UZM-6 from example 3 was calcined in $N_2$ for 1 hr and 19 hr in air at 520°C and was designated UZM-6, Ex. 3. The UZM-6 from example 17 was obtained in a proton form by calcination at 350°C for 1.5 hr, then 450°C for 1.5 hr, and 580°C for 1 hr in $N_2$, followed by 6 hr calcination in air. Then 85 g of the calcined material was exchanged in 2L of 10% $NH_4Cl$ solution at 80°C for 2 hr, which was repeated 3 times. Finally, the sample was calcined for 2 hr at 500°C in air. This sample was designated UZM-6, Ex. 17. For microreactor testing, the samples were sized to 40-60 mesh and activated in a muffle furnace in a flow of air at 550°C for 2 hr. The meshed samples, 125 mg, were loaded into an 11 mm i.d. quartz reactor sitting in a furnace; the outlet of the reactor was at atmospheric pressure. The samples were preheated to 375°C in a flow of $H_2$. The flow of $H_2$ was then directed through a saturator, where it was saturated with either m-xylene or o-xylene at 0°C. The flow of $H_2$ was controlled at 50 cc/min. The furnace was then stepped through temperatures of 375°C, 400°C, 425°C, 450°C, 475°C, and 425°C. Product effluents at each temperature were directed to an on-line GC to obtain activity and selectivity measurements. The results from m-xylene and o-xylene isomerization are shown in Tables 18a and 18b, respectively.

**Table 18A. m-Xylene Isomerization Data.**

| m-xylene isomerization | UZM-5, Ex. 1 | | | UZM-5, Ex. 1-AW | | |
|---|---|---|---|---|---|---|
| Temperature (deg C) | 375 | 425 | 475 | 375 | 425 | 475 |
| Flow Rate (cc/min) | 50 | 50 | 50 | 50 | 50 | 50 |
| | | | | | | |
| Methane | 0.05 | 0.21 | 0.23 | 0.17 | 0.22 | 0.21 |
| Benzene | 0.09 | 0.09 | 0.1 | 0.1 | 0.1 | 0.1 |
| Toluene | 0.78 | 0.57 | 0.51 | 1.18 | 0.9 | 0.92 |
| Ethylbenzene | 0 | 0 | 0 | 0 | 0 | 0 |
| p-Xylene | 20.69 | 24.27 | 24.36 | 24.12 | 24.14 | 23.97 |
| m-Xylene | 62.58 | 54.19 | 51.78 | 53.92 | 51.57 | 50.95 |
| o-Xylene | 15.06 | 20.45 | 22.77 | 19.25 | 22.15 | 22.93 |
| 135TMBZ | 0.18 | 0 | 0 | 0.31 | 0.22 | 0.21 |
| 124TMBZ | 0.5 | 0.22 | 0.24 | 0.83 | 0.6 | 0.6 |
| 123TMBZ | 0.07 | 0 | 0 | 0.11 | 0.09 | 0.1 |
| 1235TTMBZ | 0 | 0 | 0 | 0 | 0 | 0 |
| 1234TTMBZ | 0 | 0 | 0 | 0 | 0 | 0 |
| 1245TTMBZ | 0 | 0 | 0 | 0 | 0 | 0 |
| Total Unknowns | 0 | 0 | 0 | 0 | 0 | 0 |
| m-xylene conversion | 37.42 | 45.81 | 48.22 | 46.08 | 48.43 | 49.05 |

**Table 18A (cont.). m-Xylene Isomerization Data.**

| m-xylene isomerization | UZM-6, Ex. 3 | | | UZM-6, Ex. 17 | | |
|---|---|---|---|---|---|---|
| Temperature (deg C) | 375 | 425 | 475 | 375 | 425 | 475 |
| Flow Rate (cc/min) | 50 | 50 | 50 | 50 | 50 | 50 |
| | | | | | | |
| Methane | 0.12 | 0.16 | 0.18 | 0.12 | 0.32 | 0.65 |
| Benzene | 0.12 | 0.1 | 0.12 | 0.14 | 0.12 | 0.22 |
| Toluene | 8.06 | 5.53 | 5.3 | 10.57 | 6.76 | 9.08 |
| Ethylbenzene | 0 | 0 | 0 | 0 | 0 | 0 |
| p-Xylene | 19.85 | 21.31 | 21.49 | 18.41 | 20.7 | 19.21 |
| m-Xylene | 43.21 | 45.63 | 45.56 | 40.01 | 44.27 | 40.89 |
| o-Xylene | 18.09 | 19.89 | 20.55 | 16.81 | 19.33 | 18.51 |
| 135TMBZ | 2.56 | 1.75 | 1.55 | 3.35 | 2.01 | 2.51 |
| 124TMBZ | 6.57 | 4.67 | 4.33 | 8.55 | 5.37 | 6.97 |
| 123TMBZ | 0.92 | 0.7 | 0.69 | 1.2 | 0.8 | 1.11 |
| 1235TTMBZ | 0.25 | 0.14 | 0.13 | 0.4 | 0.18 | 0.3 |
| 1234TTMBZ | 0.06 | 0 | 0 | 0.09 | 0 | 0.08 |
| 1245TTMBZ | 0.2 | 0.11 | 0.1 | 0.32 | 0.13 | 0.22 |
| Total Unknowns | 0 | 0 | 0 | 0 | 0 | 0.25 |
| m-xylene conversion | 56.69 | 54.37 | 54.44 | 59.99 | 45.73 | 59.11 |

**Table 18B. o-Xylene Isomerization Data.**

| o-xylene isomerization | UZM-5, Ex. 1-AW | | | UZM-5, Ex. 2 | | |
|---|---|---|---|---|---|---|
| Temperature (deg C) | 375 | 425 | 475 | 375 | 425 | 475 |

(continued)

| o-xylene isomerization | UZM-5, Ex. 1-AW | | | UZM-5, Ex. 2 | | |
|---|---|---|---|---|---|---|
| Flow Rate (cc/min) | 50 | 50 | 50 | 50 | 50 | 50 |
| | | | | | | |
| METHANE | 0.00 | 0.00 | 0.00 | 0.00 | 0.00 | 0.00 |
| ETHANE | 0.06 | 0.07 | 0.20 | 0.09 | 0.13 | 0.19 |
| PROPANE | 0.00 | 0.00 | 0.00 | 0.00 | 0.00 | 0.00 |
| ETHYLCYCLOHEXANE | 0.11 | 0.10 | 0.07 | 0.11 | 0.09 | 0.00 |
| BENZENE | 0.13 | 0.13 | 0.13 | 0.15 | 0.15 | 0.16 |
| TOLUENE | 0.12 | 0.13 | 0.22 | 0.29 | 0.34 | 0.50 |
| ETHYLBENZENE | 0.00 | 0.00 | 0.00 | 0.12 | 0.11 | 0.10 |
| p-XYLENE | 6.18 | 14.60 | 20.61 | 14.19 | 21.04 | 23.06 |
| m-XYLENE | 30.68 | 43.71 | 49.52 | 45.15 | 50.51 | 51.16 |
| o-XYLENE | 62.55 | 41.06 | 29.00 | 39.52 | 27.19 | 24.09 |
| 135-TM-BENZENE | 0 | 0 | 0 | 0.10 | 0.12 | 0.17 |
| 124-TM-BENZENE | 0 | 0 | 0 | 0.28 | 0.31 | 0.47 |
| 123-TM-BENZENE | 0.13 | 0.19 | 0.25 | 0.00 | 0.00 | 0.08 |
| 1245-TETRAM-BENZ | 0 | 0 | 0 | 0.00 | 0.00 | 0.00 |
| 1235-TETRAM-BENZ | 0 | 0 | 0 | 0.00 | 0.00 | 0.00 |
| 1234-TETRAM-BENZ | 0 | 0 | 0 | 0.00 | 0.00 | 0.00 |
| o-xylene conversion | 37.45 | 58.84 | 71.00 | 60.48 | 72.81 | 75.91 |

## Claims

1. A microporous crystalline zeolite having a composition in the as synthesized form on an anhydrous basis in terms of mole ratios of the elements of:

$$M_m^{n+} R_r^{p+} Al_{(1-x)} E_x Si_y O_z$$

where M is at least one exchangeable cation selected from the group consisting of alkali and alkaline earth metals, "m" is the mole ratio of M to (Al + E) and varies from 0 to 1.2, R is a nitrogen-containing organic cation selected from the group consisting of protonated amines, protonated diamines, protonated alkanolamines, quaternary ammonium ions, diquaternaryammonium ions, quaternized alkanolamines and mixtures thereof, "r" is the mole ratio of R to (Al + E) and has a value of 0.25 to 3.0, E is at least one element selected from the group consisting of Ga, Fe, Cr, In and B, "x" is the mole fraction of E and varies from 0 to 0.5, "n" is the weighted average valence of M and has a value of +1 to +2, "p" is the weighted average valence of R and has a value of +1 to +2, "y" is the mole ratio of Si to (Al + E) and varies from 5 to 12 and "z" is the mole ratio of O to (Al + E) and has a value determined by the equation:

$$z = (m \bullet n + r \bullet p + 3 + 4 \bullet y)/2$$

the zeolite **characterized in that** it has at least two x-ray diffraction peaks, one at a *d*-spacings of 3.9±0.12Å and one at a *d*-spacing of 8.8±0.20Å.

**2.** The zeolite of claim 1 **characterized in that** it has a x-ray powder diffraction pattern which contains at least the *d*-spacings and relative intensities of one of Tables A to C.

**3.** The zeolite of claims 1 or 2 where M is at least one metal selected from the group consisting of lithium, cesium, sodium, potassium, strontium, barium, calcium, magnesium and R is a quaternary ammonium cation.

**4.** A process for preparing the microporous crystalline zeolite of any of claims 1-3 which comprises forming a reaction mixture containing reactive sources of R, Al, Si and optionally E and/or M and reacting the reaction mixture at reaction conditions which include a temperature of 100°C to 175°C for a period of 12 hr to 2 weeks, the reaction mixture having a composition expressed in terms of mole ratios of the oxides of:

$$aM_{2/n}O:bR_{2/p}O:(1-c)Al_2O_3:cE_2O_3:dSiO_2:eH_2O$$

where "a" has a value of 0 to 2, "b" has a value of 1.5 to 30, "c" has a value of 0 to 0.5, "d" has a value of 5 to 30, and "e" has a value of 30 to 6000.

**5.** The process of claim 4 where the source of E is selected from the group consisting of alkali borates, boric acid, precipitated gallium oxyhydroxide, gallium sulfate, ferric sulfate, ferric chloride, chromium chloride, chromium nitrate, indium chloride and indium nitrate.

**6.** The process of claim 4 where the aluminum source is selected from the group consisting of aluminum isopropoxide, aluminum sec-butoxide, precipitated alumina, $Al(OH)_3$, aluminum metal and aluminum salts.

**7.** The process of claim 4 where the reaction conditions include a temperature of 140°C to 160°C for a period of 2 days to 5 days.

**8.** The use of the microporous crystalline zeolite of any of claims 1-3 in a hydrocarbon conversion process comprising contacting a hydrocarbon stream with the microporous crystalline zeolite at hydrocarbon conversion conditions to give a converted product.

**9.** A process for the isomerization of a non-equilibrium feed mixture comprising xylenes and ethylbenzene comprising contacting the feed mixture in the presence of hydrogen in an isomerization zone with a catalyst composite comprising an effective amount of at least one platinum-group metal component and the crystalline aluminosilicate zeolite of any of claims 1-3 at isomerization conditions to obtain an isomerized product comprising a higher proportion of p-xylene than in the feed mixture.

**10.** The process of Claim 9 wherein the platinum-group metal component is present in an amount from 0.01 to 5 mass-% on an elemental basis.

**11.** The process of Claim 9 wherein ortho-xylene is recovered from one or both of the isomerized product and fresh feed mixture.

**12.** The process of Claim 9 further comprising recovery of para-xylene by selective adsorption from the isomerized product and a fresh feed mixture.

**13.** The process of claim 9 where the isomerization conditions include a temperature of 100°C to 500°C, a pressure from 1 to 50 atmospheres and a liquid hourly spare velocity of 0.5 to $10hr^{-1}$.

**14.** A process for monoalkylating aromatic compounds comprising reacting under alkylation conditions an olefin with an alkylatable aromatic compound to provide an alkylated compound in the presence of a catalyst comprising the crystalline aluminosilicate zeolite of any of claims 1-3.

**15.** The process of Claim 14 where the olefin contains from 2 up to 20 carbon atoms.

**16.** The process of claim 14 where the process is carried out under at least partial liquid phase conditions.

**17.** A process for preparing cumene by the alkylation of benzene with propylene comprising reacting propylene with benzene at a temperature between 90°C and 200°C at a pressure sufficient to maintain at least a partial liquid phase

in the presence of a catalyst comprising the crystalline aluminosilicate zeolite of any of claims 1-3.

**18.** A transalkylation process comprising reacting under transalkylation reaction conditions a polyalkylated aromatic compound with a nonalkylated aromatic compound, wherein at least one alkyl group is transferred from the polyalkylated aromatic compound to the nonalkylated aromatic compound in the presence of the microporous crystalline zeolite of any of claims 1-3.

## Patentansprüche

**1.** Mikroporöser kristalliner Zeolith, welcher in der auf nicht-wässriger Basis synthetisierten Form die folgenden Elemente in nachstehenden Molverhältnissen enthält:

$$M_m^{n+}R_r^{p+}Al_{(1-x)}E_xSi_yO_z,$$

worin bedeuten: M bedeutet mindestens ein austauschbares Kation, ausgewählt aus der Gruppe, bestehend aus Alkali- und Erdalkalimetallen, "m" bedeutet das Molverhältnis von M zu (Al + E) und variiert von 0 bis 1,2, R bedeutet ein stickstoffhaltiges organisches Kation, ausgewählt aus der Gruppe, bestehend aus protonierten Aminen, protonierten Diaminen, protonierten Alkanolaminen, quaternären Ammoniumionen, diquaternären Ammoniumionen, quaternisierten Alkanolaminen und Gemischen davon, "r" bedeutet das Molverhältnis von R zu (Al + E) und hat einen Wert von 0,25 bis 3,0, E bedeutet mindestens ein Element, ausgewählt aus der Gruppe, bestehend aus Ga, Fe, Cr, In und B, "x" bedeutet den Molanteil von E und variiert von 0 bis 0,5, "n" bedeutet die gewichtete durchschnittliche Wertigkeit von M und hat einen Wert von +1 bis +2, "p" bedeutet die gewichtete durchschnittliche Wertigkeit von R und hat einen Wert von +1 bis +2, "y" bedeutet das Molverhältnis von Si zu (Al + E) und variiert von 5 bis 12, und "z" bedeutet das Molverhältnis von O zu (Al + E) und hat einen Wert, der bestimmt ist durch die Gleichung

$$z = (m \cdot n + r \cdot p + 3 + 4 \cdot y)/2;$$

der Zeolith ist **dadurch gekennzeichnet, dass** er mindestens zwei Röntgenbeugungspeaks aufweist, einen bei einem d-Abstand von 3,9±0,12Å, und einen bei einem d-Abstand von 8,6±0,20Å.

**2.** Zeolith nach Anspruch 1, **dadurch gekennzeichnet, dass** er ein Pulver-Röntgenbeugungsmuster aufweist, welches mindestens die d-Abstände und relativen Intensitäten aus einer der Tabellen A bis C enthält.

**3.** Zeolith nach Anspruch 1 oder 2, worin M mindestens ein Metall, ausgewählt aus der Gruppe, bestehend aus Lithium, Cäsium, Natrium, Kalium, Strontium, Barium, Calcium, Magnesium, und R ein quaternäres Ammoniumkation bedeutet.

**4.** Verfahren zur Herstellung des mikroporösen kristallinen Zeolithen nach einem der Ansprüche 1 bis 3, umfassend die Bildung eines Reaktionsgemisches, enthaltend reaktive Quellen von R, Al, Si und gegebenenfalls von E und/oder M, und Umsetzen des Reaktionsgemisches bei Reaktionsbedingungen, die eine Temperatur von 100°C bis 175°C über einen Zeitraum von 12 Stunden bis 2 Wochen umfassen, wobei das Reaktionsgemisch eine Zusammensetzung aufweist, die ausgedrückt werden kann als Molverhältnisse der Oxide von:

$$aM_{2n}O: bR_{2/p}O: (1\text{-}c)\ Al_2O_3: cE_2O_3: dSiO_2: eH_2O,$$

worin "a" einen Wert von 0 bis 2, "b" einen Wert von 1,5 bis 30, "c" einen Wert von 0 bis 0,5, "d" einen Wert von 5 bis 30 und "e" einen Wert von 30 bis 6.000 hat.

**5.** Verfahren nach Anspruch 4, worin die Quelle von E aus der Gruppe, bestehend aus Alkaliboraten, Borsäure, gefälltem Galliumoxyhydroxid, Galliumsulfat, Eisen(III)-sulfat, Eisen(III)-chlorid, Chromchlorid, Chromnitrat, Indiumchlorid und Indiumnitrat ausgewählt ist.

**6.** Verfahren nach Anspruch 4, worin die Aluminiumquelle aus der Gruppe, bestehend aus Aluminiumisopropoxid,

Aluminium-sek.-butoxid, gefälltem Aluminiumoxid, $Al(OH)_3$, metallischem Aluminium und Aluminiumsalzen ausgewählt ist.

**7.** Verfahren nach Anspruch 4, worin die Reaktionsbedingungen eine Temperatur von 140°C bis 160°C über einen Zeitraum von 2 Tagen bis 5 Tagen umfassen.

**8.** Verwendung des mikroporösen kristallinen Zeolithen nach einem der Ansprüche 1 bis 3 bei einem Kohlenwasserstoff-Konvertierungsverfahren, umfassend das Kontaktieren eines Kohlenwasserstoffstroms mit dem mikroporösen kristallinen Zeolith bei Kohlenwasserstoff-Konvertierungsbedingungen, um ein konvertiertes Produkt zu erhalten.

**9.** Verfahren zur Isomerisierung eines sich nicht im Gleichgewicht befindlichen Zufuhrgemisches, enthaltend Xylole und Ethylbenzol, umfassend das Kontaktieren des Zufuhrgemisches in Anwesenheit von Wasserstoff in einer Isomerisierungszone mit einem Katalysatorgemisch, enthaltend eine wirksame Menge mindestens einer Metallkomponente aus der Platingruppe und dem kristallinen Aluminosilikat-Zeolith nach einem der Ansprüche 1 bis 3 bei Isomerisierungsbedingungen, um ein isomerisiertes Produkt zu erhalten, welches einen höheren Anteil an p-Xylol als das Zufuhrgemisch aufweist.

**10.** Verfahren nach Anspruch 9, worin die Metallkomponente der Platingruppe in einer Menge von 0,01 bis 5 Massen-% auf elementarer Basis vorhanden ist.

**11.** Verfahren nach Anspruch 9, worin das ortho-Xylol entweder aus dem isomerisierten Produkt oder dem frischen Zufuhrgemisch oder aus beiden gewonnen wird.

**12.** Verfahren nach Anspruch 9, weiterhin umfassend die Gewinnung von para-Xylol durch selektive Adsorption aus dem isomerisierten Produkt und einem frischen Zufuhrgemisch.

**13.** Verfahren nach Anspruch 9, worin die Isomierisierungsbedingungen eine Temperatur von 100°C bis 500°C, einen Druck von 1 bis 50 Atmosphären und eine stündliche Flüssigkeits-Raumgeschwindigkeit (liquid hourly space velocity) von 0,5 bis 10/h umfassen.

**14.** Verfahren zur Monoalkylierung von aromatischen Verbindungen, umfassend die Umsetzung eines Olefins mit einer alkylierbaren aromatischen Verbindung unter Alkylierungsbedingungen in Anwesenheit eines Katalysators, enthaltend den kristallinen Aluminosilikat-Zeolith nach einem der Ansprüche 1 bis 3, um eine alkylierte Verbindung zu erzeugen.

**15.** Verfahren nach Anspruch 14, worin das Olefin 2 bis 20 Kohlenstoffatome enthält.

**16.** Verfahren nach Anspruch 14, worin das Verfahren zumindest teilweise in der Flüssigphase durchgeführt wird.

**17.** Verfahren zur Herstellung von Cumol durch Alkylierung von Benzol mit Propylen, umfassend die Umsetzung von Propylen mit Benzol bei einer Temperatur zwischen 90°C und 200°C bei einem Druck, der ausreicht, um zumindest teilweise eine flüssige Phase aufrecht zu erhalten, in Anwesenheit eines Katalysators, enthaltend den kristallinen Aluminosilikat-Zeolith nach einem der Ansprüche 1 bis 3.

**18.** Transalkylierungsverfahren, umfassend die Umsetzung einer polyalkylierten aromatischen Verbindung mit einer nichtalkylierten aromatischen Verbindung unter Transalkylierungsbedingungen in Anwesenheit des mikroporösen kristallinen Zeolithen nach einem der Ansprüche 1 bis 3, wobei mindestens eine Alkylgruppe von der polyalkylierten aromatischen Verbindung auf die nichtalkylierte aromatische Verbindung übertragen wird.

## Revendications

**1.** Zéolite cristalline microporeuse ayant une composition, sous la forme synthétisée, sur une base anhydre en termes de rapports molaires des éléments, de :

$$M_m^{n+} R_r^{p+} Al_{(1-x)} E_x Si_y O_z$$

où M est au moins un cation échangeable choisi dans l'ensemble constitué de métaux alcalins et alcalino-terreux, "m" est le rapport molaire de M à (Al + E) et varie de 0 à 1,2, R est un cation organique contenant de l'azote, choisi dans l'ensemble constitué d'amines protonées, de diamines protonées, d'alcanolamines protonées, d'ions ammonium quaternaire, d'ions ammonium diquaternaire, d'alcanolamines quaternisées et de mélanges de ceux-ci, "r" est le rapport molaire de R à (A1 + E) et a une valeur de 0,25 à 3,0, E est au moins un élément choisi dans l'ensemble constitué de Ga, Fe, Cr, In et B, "x" est la fraction molaire de E et varie de 0 à 0,5, "n" est la valence moyenne pesée de M et a une valeur de +1 à +2, "p" est la valence moyenne pesée de R et a une valeur de +1 à +2, "y" est le rapport molaire de Si à (Al + E) et varie de 5 à 12, et "z" est le rapport molaire de O à (Al + E) et a une valeur déterminée au moyen de l'équation :

$$z = (m \cdot n + r \cdot p + 3 + 4 \cdot y)/2$$

la zéolite étant **caractérisée en ce qu'**elle a au moins deux pics de diffraction des rayons X, l'un à un espacement d de 3,9 ± 0,12 Å et l'un à un espacement d de 8,6 ± 0,20 Å.

**2.** Zéolite selon la revendication 1, **caractérisée en ce qu'**elle a un motif de diffraction de poudre aux rayons X qui contient au moins les espacements *d* et les intensités relatives de l'un des tableaux A à C.

**3.** Zéolite selon la revendication 1 ou 2, dans laquelle M est au moins un métal choisi dans l'ensemble constitué du lithium, du césium, du sodium, du potassium, du strontium, du baryum, du calcium, du magnésium, et R est un cation ammonium quaternaire.

**4.** Procédé de préparation de la zéolite cristalline microporeuse selon l'une quelconque des revendications 1 à 3, comprenant la formation d'un mélange réactionnel contenant des sources réactives de R, Al, Si et éventuellement E et/ou M, et la mise en réaction du mélange réactionnel dans des conditions de réaction qui comprennent une température de 100°C à 175°C pendant une durée de 12 h à 2 semaines, le mélange réactionnel ayant une composition, exprimée en termes de rapports molaires des oxydes, de :

$$aM_{2/n}O{:}bR_{2/}pO{:}\ (1\text{-}c)\ Al_2O_3 : cE_2O_3 : dSiO_2 : eH_2O$$

où "a" a une valeur de 0 à 2, "b" a une valeur de 1,5 à 30, "c" a une valeur de 0 à 0,5, "d" a une valeur de 5 à 30 et "e" a une valeur de 30 à 6000.

**5.** Procédé selon la revendication 4, dans lequel la source de E est choisie dans l'ensemble constitué de borates alcalins, de l'acide borique, d'oxyhydroxyde de gallium précipité, de sulfate de gallium, de sulfate ferrique, de chlorure ferrique, de chlorure de chrome, de nitrate de chrome, de chlorure d'indium et de nitrate d'indium.

**6.** Procédé selon la revendication 4, dans lequel la source d'aluminium est choisie dans l'ensemble constitué de l'isopropylate d'aluminium, du sec.-butylate d'aluminium, d'alumine précipitée, d'$Al(OH)_3$, de métal aluminium et de sels d'aluminium.

**7.** Procédé selon la revendication 4, dans lequel les conditions de réaction comprennent une température de 140°C à 160°C pendant une durée de 2 jours à 5 jours.

**8.** Utilisation de la zéolite cristalline microporeuse selon l'une quelconque des revendications 1 à 3 dans un procédé de transformation d'hydrocarbures, comprenant l'amenée d'un flux d'hydrocarbures au contact de la zéolite cristalline microporeuse dans des conditions de transformation d'hydrocarbures de façon à donner un produit transformé.

**9.** Procédé d'isomérisation d'un mélange d'amenée qui n'est pas à l'équilibre, comprenant des xylènes et de l'éthylbenzène, comprenant la mise en contact du mélange d'amenée, en présence d'hydrogène, dans une zone d'isomérisation, avec un composite de catalyseur comprenant une quantité efficace d'au moins un composant métallique

du groupe du platine et la zéolite cristalline à base d'aluminosilicate selon l'une quelconque des revendications 1 à 3 dans des conditions d'isomérisation pour obtenir un produit isomérisé comprenant une proportion de p-xylène supérieure à celle présente dans le mélange d'amenée.

**10.** Procédé selon la revendication 9, dans lequel le composant métallique du groupe du platine est présent en une quantité de 0,01 à 5 % en poids sur la base des éléments.

**11.** Procédé selon la revendication 9, dans lequel on recueille de l'ortho-xylène à partir de l'un ou des deux du produit isomérisé et du mélange d'amenée frais.

**12.** Procédé selon la revendication 9, comprenant en outre la récupération de para-xylène par adsorption sélective à partir du produit isomérisé et d'un mélange d'amenée frais.

**13.** Procédé selon la revendication 9, dans lequel les conditions d'isomérisation comprennent une température de 100°C à 500°C, une pression de 1 à 50 atmosphères et une vitesse spatiale horaire de liquide de 0,5 à 10 $h^{-1}$.

**14.** Procédé de monoalkylation de composés aromatiques, comprenant la mise en réaction, dans des conditions d'alkylation, d'une oléfine avec un composé aromatique pouvant être alkylé pour fournir un composé alkylé en présence d'un catalyseur comprenant la zéolite cristalline à base d'aluminosilicate selon l'une quelconque des revendications 1 à 3.

**15.** Procédé selon la revendication 14, dans lequel l'oléfine contient de 2 jusqu'à 20 atomes de carbone.

**16.** Procédé selon la revendication 14, ledit procédé étant mis en oeuvre dans des conditions de phase au moins partiellement liquide.

**17.** Procédé de préparation de cumène par alkylation de benzène avec du propylène, comprenant la mise en réaction de propylène avec du benzène à une température comprise entre 90°C et 200°C et à une pression suffisante pour maintenir au moins une phase liquide partielle en présence d'un catalyseur comprenant la zéolite cristalline à base d'aluminosilicate selon l'une quelconque des revendications 1 à 3.

**18.** Procédé de transalkylation comprenant la mise en réaction, dans des conditions de réaction de transalkylation, d'un composé aromatique polyalkylé avec un composé aromatique non alkylé, dans lequel au moins un groupe alkyle est transféré du composé aromatique polyalkylé au composé aromatique non alkylé en présence de la zéolite cristalline microporeuse selon l'une quelconque des revendications 1 à 3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5015796 A
- US 2620314 A
- US 3201491 A
- US 3626020 A
- US 3696107 A
- US 4039599 A
- US 4184943 A
- US 4381419 A
- US 4402832 A


### Non-patent literature cited in the description


- Atlas of Zeolite Structure Types. International Zeolite Association (IZA
- **D.W. BRECK.** Zeolite Molecular Sieves. John Wiley and Sons, 1974, 636
- **H. PINES.** THE CHEMISTRY OF CATALYTIC HYDROCARBON CONVERSIONS. Academic Press, 1981, 123-154